# FASCICULE DE BREVET EUROPEEN

(11) **EP 4 058 133 B1**
(45) Date de publication et mention de la délivrance du brevet: **06.08.2025**
(21) Numéro de dépôt: 20861964.3
(22) Date de dépôt: 13.11.2020
(51) Int. Cl.: A61N 1/39, A61N 1/365, A61N 1/362

(54) **STIMULATEUR CARDIAQUE ET DEFIBRILLATEUR CARDIAQUE SOUS CUTANE**
SUBKUTANER HERZSCHRITTMACHER UND HERZSCHRITTMACHER
SUBCUTANEOUS CARDIAC DEFIBRILLATOR AND CARDIAC PACEMAKER

(30) Priorité: 13.11.2019 FR 1912662
(43) Date de publication de la demande: 21.09.2022
(73) Titulaire: Le Franc, Pierre, 76000 Rouen (FR)
(72) Inventeur: Le Franc, Pierre, 76000 Rouen (FR)
(74) Mandataire: Cabinet Beau de Loménie
(86) Numéro de dépôt international: PCT/FR2020/052074
(87) Numéro de publication internationale: WO 2021/094692

(56) Documents cités:
- WO-A1-01/36046
- US-A1- 2004 220 623
- US-A1- 2018 056 079
- US-B2- 10 238 883
- US-B2- 9 511 233

## Description

### Technique antérieure

L'invention est telle que définie dans les revendications annexées à la présente description, et se rapporte au domaine général des dispositifs médicaux. Elle concerne plus particulièrement le traitement d'anomalies du rythme cardiaque.

Dans cette section, nous décrivons la structure et l'activité électrique normale du cœur, des pathologies du cœur liées à des anomalies de son activité électrique, les solutions connues de l'état de la technique pour traiter ces pathologies, et les limites de ces solutions.

### Structure et activité électrique du cœur :

Le cœur se compose de deux compartiments, droit et gauche, dont chacun comporte deux cavités: une oreillette et un ventricule.

La succession des battements du cœur est cyclique. Chaque battement correspond à une même séquence comme suit :
- le sang pauvre en oxygène arrive au cœur dans l'oreillette droite en provenance des autres organes du corps (à l'exception des poumons) par le réseau veineux.
- le sang est chassé vers le ventricule droit par une contraction de l'oreillette droite.
- ensuite, les ventricules se contractent. Cette contraction des ventricules propulse à son tour le sang du ventricule droit vers les poumons où il va se charger en oxygène.
- de retour au cœur par les veines pulmonaires, le sang riche en oxygène s'accumule dans l'oreillette gauche.
- lors de la contraction de l'oreillette gauche, le sang passe dans le ventricule gauche.
- ensuite, le ventricule gauche se contracte et le sang est envoyé vers les autres organes, à l'exception des poumons par le réseau artériel.

Le transport du sang du cœur vers les poumons et vice-versa est appelé circulation pulmonaire, ou petite circulation. Le transport du sang du cœur vers les autres organes du corps, à l'exception des poumons, et vice-versa est appelé circulation systémique, ou grande circulation.

Les deux oreillettes se contractent au même temps, et les deux ventricules se contractent au même temps. La contraction des oreillettes se fait lorsque les ventricules sont relâchés, et le relâchement des oreillettes est synchrone avec la contraction des ventricules.

La phase de contraction des oreillettes (ou des ventricules) permet d'évacuer le sang des cavités auriculaires (ou ventriculaires), tandis que la phase de relâchement des oreillettes (ou des ventricules) permet le remplissage sanguin dans les cavités auriculaires (ou ventriculaires).

La contraction du tissu musculaire du cœur est provoquée par la propagation d'une impulsion électrique le long des fibres musculaires cardiaques.

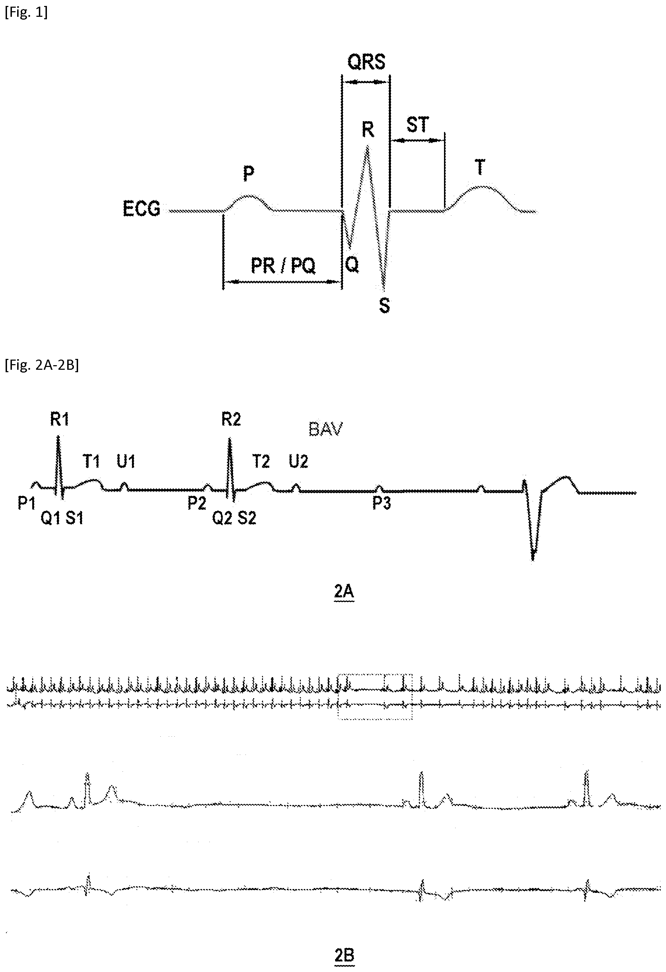

L'activité électrique du cœur est en concordance avec son battement. La **figure 1** illustre un cycle de l'activité électrique normale du cœur. La représentation graphique de l'activité électrique du cœur est appelée une électrocardiographie. Nous notons ici par l'activité électrique « normale » du cœur, l'activité d'un cœur sain, qui ne présente pas d'anomalies pathologiques. Le cycle comporte des phases appelées:
- onde P, marquant la contraction des oreillettes et leur dépolarisation électrique ;
- intervalle PR appelé aussi intervalle PQ, correspondant au temps de conduction auriculo-ventriculaire ;
- complexe QRS, correspondant à la dépolarisation et à la contraction des ventricules, le complexe QRS masque la repolarisation électrique et le relâchement des oreillettes, qui se produisent pendant la contraction des ventricules ;
- segment isoélectrique ST, correspondant à une phase de contraction isovolumétrique (temps de chasse ventriculaire) pendant laquelle il n'y a pas de propagation électrique dans le muscle cardiaque; et
- onde T correspondant à la repolarisation et au relâchement ventriculaire. La repolarisation désigne le retour à l'état électrique de repos. Une onde U, non représentée sur la figure1, peut prolonger l'onde T en cas de repolarisation ventriculaire lente.

### Des pathologies rythmiques du cœur :

On distingue deux types de pathologies rythmiques du cœur :
- les bradycardies : ralentissement anormal du rythme ; et
- les tachycardies : accélération anormale du rythme.
Pour chacun de ces deux types, on distingue plusieurs formes de pathologies.

Les **figures 2A et 2B** illustrent une activité électrique anormale de cœurs atteints d'une pathologie de type « bradycardie ».

L'électrocardiographie de la figure 2A montre deux cycles normaux, de P1 à U1, et de P2 à U2, suivis d'un cycle ne comportant qu'une onde P3. La bradycardie est détectée par une absence du complexe QRS, la contraction des ventricules n'a donc pas eu lieu. Dans cet exemple, la séquence des ondes P est normale, témoignant d'une activité auriculaire normale. Cette bradycardie correspond à un trouble de la conduction électrique entre oreillettes et ventricules. Pour remédier à ce problème, il est nécessaire de stimuler électriquement le ventricule droit, voire les deux ventricules, après la détection d'une onde P, pour que les ventricules se contractent et pour que le cœur reprenne son cycle normal. La puissance électrique nécessaire pour une stimulation cardiaque est généralement comprise entre 0.5 à 2.5 Volt.

La figure 2B illustre un autre type de bradycardie. Dans cet exemple, l'activité auriculaire est absente, tandis que les ventricules se contractent de façon autonome. Il s'agit d'une pathologie rythmique auriculaire appelée également dysfonction sinusale. Pour remédier à ce problème, il est nécessaire de stimuler l'oreillette pour déclencher sa contraction, et générer une conduction et une contraction ventriculaire à la suite de l'activation de l'oreillette.

A noter que ces deux types de bradycardie peuvent être associés, nécessitant alors une double stimulation des oreillettes et des ventricules.

Il existe une autre famille de pathologies cardiaques qui sont les tachycardies. Les tachycardies peuvent avoir leur origine à trois étages: dans les oreillettes (telles que la fibrillation auriculaire et la tachycardie atriale focale ou flutter), dans les ventricules (telles que la tachycardie ventriculaire ou la fibrillation ventriculaire), ou à la jonction entre oreillettes et ventricules. Nous décrivons ici les tachycardies naissant dans les ventricules.

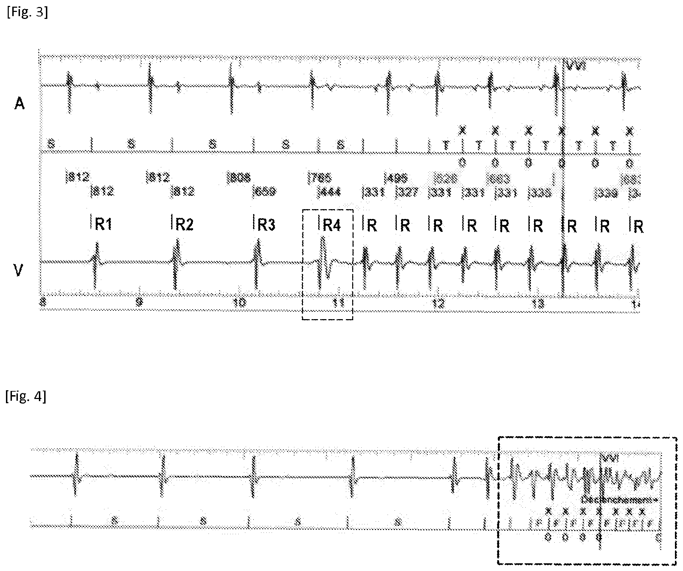

La **figure 3** illustre une activité électrique anormale d'un cœur atteint d'une pathologie de type « tachycardie ventriculaire ». L'électrocardiographie de la figure 3 comporte deux lignes marquées par A (pour « atria » en anglais) et V (pour « ventricle » en anglais), et correspondant à des enregistrements au niveau des oreillettes, et des ventricules respectivement. A partir du quatrième complexe noté par R4, Le rythme de contraction et de relâchement des ventricules reste régulier, mais s'accélère brutalement et se désynchronise du rythme électrique des oreillettes. Il s'agit d'une tachycardie ventriculaire, naissant dans les ventricules. La tachycardie ventriculaire présente deux risques majeurs : un mauvais fonctionnement cardiaque générant une insuffisance cardiaque d'autant plus rapide que le cœur est fatigué et un risque de dégénérer en fibrillation ventriculaire, pouvant résulter en un arrêt cardiaque. Pour traiter cette pathologie, il est nécessaire de stimuler électriquement au moins l'un des ventricules, avec une stimulation électrique, nommée stimulation anti tachycardie ATP (pour « Anti Tachycardia Pacing » en anglais), d'une fréquence plus élevée que celle de la tachycardie. En cas d'échec de la stimulation anti tachycardie, un choc électrique peut être nécessaire pour régulariser le cœur.

La **figure 4** illustre une activité électrique anormale d'un cœur atteint d'une pathologie de type « fibrillation ventriculaire ». A la fin de l'électrocardiogramme de la figure 4, l'activité électrique du cœur est désorganisée, sans cycle régulier. L'activité mécanique du cœur est abolie. La fibrillation présente un risque vital qu'il faut traiter dans un délai de deux minutes par une défibrillation cardiaque. La défibrillation consiste à délivrer un choc électrique au cœur, à haute énergie, de l'ordre de 800 Volt.

### Les solutions connues de l'état de la technique :

Conformément à l'état de la technique, des dispositifs médicaux peuvent être implantés dans le corps pour surveiller l'activité électrique du cœur et intervenir rapidement en cas de détection d'une arythmie cardiaque, par stimulation et/ou défibrillation cardiaque.

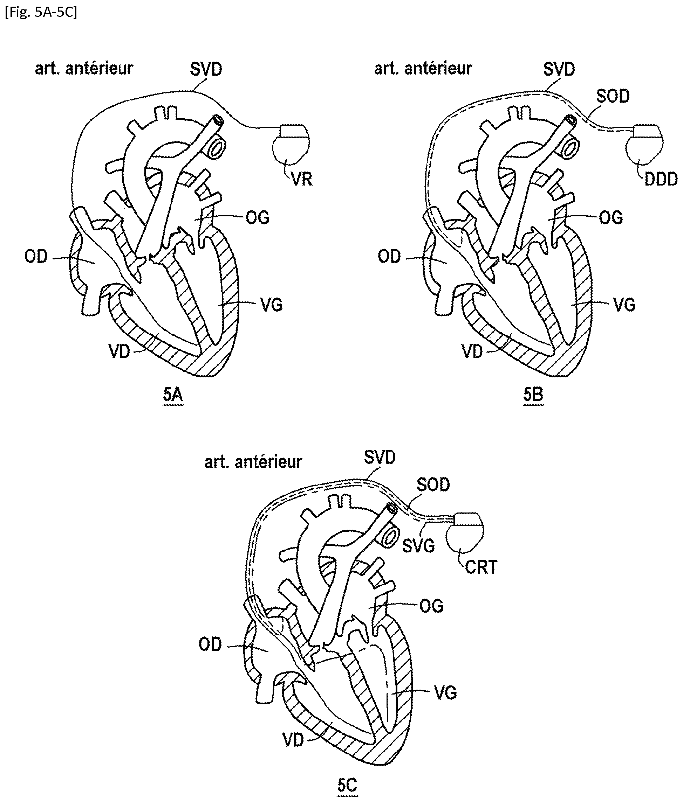

Les **figures 5A à 5C** illustrent des stimulateurs cardiaques avec sondes de l'art antérieur. Ces stimulateurs s'implantent au niveau de la poitrine, dans la région de l'épaule gauche, près de la clavicule, et comportent un boitier et au moins une sonde qui passe par une veine à l'intérieur du cœur reliant le boitier à une cavité du cœur. Le boitier comporte une source d'énergie électrique et un module de recueil et de traitement de données. La ou les sondes permettent de conduire l'information électrique du muscle au module de recueil et de traitement, et de conduire le cas échéant une réponse électrique du boitier au muscle cardiaque. Le stimulateur de type VVI ou VR (pour « Ventricle Rate response » en anglais) de la figure 5A, dit stimulateur mono-chambre ventriculaire, comporte une seule sonde SVD connectée au muscle du ventricule droit VD. Le stimulateur de type DDD ou DR (pour « Dual Rate response » en anglais) de la figure 5B, dit stimulateur double chambre, comporte en outre une deuxième sonde SOD connectée à l'oreillette droite OD. Le stimulateur de type CRT (pour « Cardiac Resynchronization Therapy » en anglais) de la figure 5C, dit stimulateur triple chambre, comporte en outre une troisième sonde SVG connectée au ventricule gauche VG. Il existe aussi des stimulateurs à quatre sondes pouvant stimuler en plus l'oreillette gauche OG.

Le rôle d'un stimulateur cardiaque est de surveiller de façon continue le rythme cardiaque en détectant l'activité électrique au contact du muscle. Lorsque l'activité reste au-dessus d'un seuil de fréquence donné, le stimulateur reste en veille et poursuit la surveillance. Lorsque la fréquence spontanée du cœur est inférieure au seuil de fréquence prédéfini, le stimulateur envoie dans la cavité défaillante des impulsions électriques brèves à une fréquence prévue pour déclencher des contractions de la cavité défaillante. Un stimulateur cardiaque permet donc de traiter les bradycardies mais ne permet ni de traiter les tachycardies ni d'effectuer une défibrillation cardiaque.

Les stimulateurs avec sondes, tels que les stimulateurs VR, DR et CRT illustrés aux figures 5Aà 5C, présentent un risque d'infection lié aux sondes. Lors de l'usure ou de la défaillance d'un tel stimulateur, une ré-intervention est nécessaire, avec un risque accru d'infection.

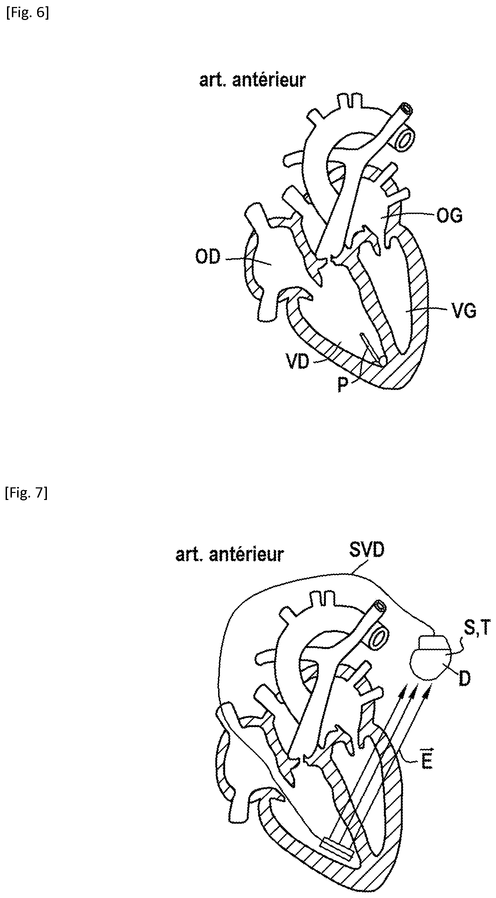

Il existe un autre type de stimulateurs cardiaques : les stimulateurs intracardiaques. La **figure 6** illustre un stimulateur cardiaque implanté à l'intérieur du ventricule droit. Il s'agit d'une capsule P autonome fixée sur la paroi de l'endocarde du ventricule droit, sans sonde ni aucune liaison avec la peau.

Dans l'état actuel de la technique, les stimulateurs intracardiaques ne peuvent être implantés que dans le ventricule droit, ils sont appelés aussi des stimulateurs endoventriculaires. Ces stimulateurs ne permettent pas de stimuler les oreillettes ni le ventricule gauche. Ce type de stimulateur ne permet pas non plus d'effectuer une défibrillation cardiaque.

Une fois installé dans le cœur, le stimulateur intracardiaque P est progressivement recouvert d'endothélium et devient difficilement accessible à une réintervention. Contrairement aux stimulateurs avec sondes VR, DR et CRT pour lesquels on peut remplacer le boitier sous cutané en conservant les sondes, il est difficile de retirer le stimulateur endocavitaire P du cœur. De ce fait, en cas de défaillance ou d'usure du stimulateur endocavitaire P, il n'est pas possible de le remplacer, mais un autre stimulateur est implanté dans le cœur en plus du stimulateur défectueux. Nous notons que cette solution présente à terme un risque de dysfonction du cœur, notamment du ventricule droit, à cause de la masse des stimulateurs endocavitaires implantés.

La **figure 7** illustre un défibrillateur transveineux D, appelé également « défibrillateur conventionnel ». Ce défibrillateur comporte un boitier métallique qui s'implante au niveau de la poitrine, dans la région de l'épaule gauche, et une sonde endocavitaire SVD' de défibrillation connectée via une veine au ventricule droit, comme l'implantation d'un stimulateur avec sonde. Le défibrillateur transveineux D comporte par ailleurs un module d'alimentation électrique de haute tension avec des condensateurs pouvant être chargés à plus de 800 Volts.

Les défibrillateurs transveineux sont dédiés au traitement des tachycardies ventriculaires et des fibrillations ventriculaires. Ils comportent en outre une fonction de stimulateur cardiaque qui permet de prendre en charge les bradycardies.

La sonde de défibrillation SVD' comporte :
- deux électrodes de basse tension pour la détection de l'activité électrique cardiaque et pour la stimulation cardiaque ; et
- au moins une électrode de haute tension pour la délivrance d'un choc électrique, cette électrode de grande surface d'échange se présente sous la forme d'une bobine métallique enroulée autour du corps de la sonde à proximité de son extrémité ventriculaire, la sonde de défibrillation SVD' peut comporter une seconde bobine, constituant une électrode de haute tension, au milieu du corps de sonde.

Comme un stimulateur avec sonde, le défibrillateur transveineux D peut comporter en outre des sondes dédiées à la stimulation cardiaque, telles qu'une deuxième sonde connectée à l'oreillette droite (défibrillateur double chambre), une troisième sonde connectée au ventricule gauche (défibrillateur triple chambre ou CRT-D) et une quatrième sonde connectée à l'oreillette gauche (défibrillateur à quatre chambres), ces sondes dédiées à la stimulation ne comportant pas d'électrode haute tension.

Le défibrillateur transveineux surveille de façon continue l'activité électrique du cœur.

Le défibrillateur transveineux D assure une fonction de stimulation cardiaque, identique à celle des stimulateurs décrits en référence aux figures 5A à 5C.

Le défibrillateur transveineux D assure en plus une fonction de défibrillation cardiaque : il détecte et traite les arythmies ventriculaires. Une tachycardie ventriculaire est définie par une fréquence cardiaque spontanée dépassant une fréquence seuil déterminée, par exemple une fréquence seuil de 170 bpm (pour « battements par minute »). Une fibrillation ventriculaire est définie par une fréquence cardiaque spontanée dépassant une fréquence seuil déterminée, par exemple de 220 bpm.

En cas de détection d'une tachycardie ventriculaire, le défibrillateur D peut traiter l'arythmie de deux façons en fonction de sa programmation:
- par une stimulation anti tachycardie ATP : une stimulation ventriculaire rapide délivrée via une sonde de stimulation, d'une fréquence supérieure à la fréquence de la tachycardie. Le défibrillateur peut délivrer un train de 8 à 20 stimulations. A la fin de ce traitement, le défibrillateur analyse à nouveau le rythme cardiaque. Le défibrillateur D peut être programmé pour délivrer plusieurs salves de stimulation successives d'agressivité croissante.
- en cas d'échec de la stimulation ATP, le défibrillateur peut délivrer un choc électrique de cardioversion entre la bobine de la sonde de défibrillation SVD' et le boitier métallique. Les documents WO 01/36046A1 et US 2004/0220623A1 décrivent des exemples de défibrillateurs transveineux.

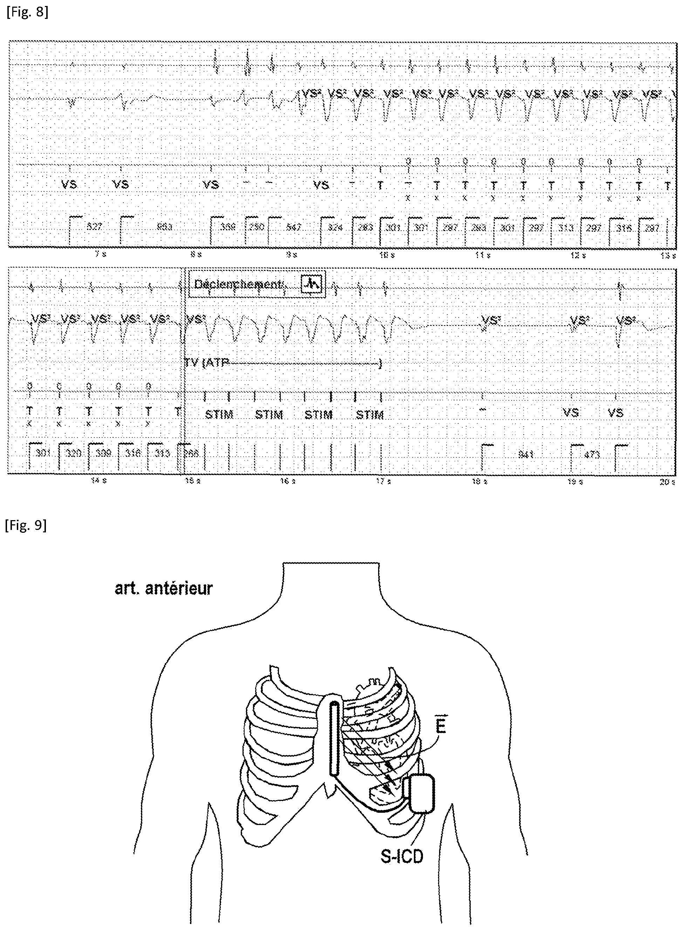

La **figure 8** est une électrocardiographie illustrant une tachycardie ventriculaire interrompue par une salve de stimulation ATP, effectuée par un défibrillateur transveineux de l'art antérieur.

En cas de fibrillation ventriculaire, le défibrillateur D charge ses condensateurs. Pendant le temps de charge, il délivre une salve unique de stimulation ATP. Une fois les condensateurs chargés, le défibrillateur vérifie la permanence de l'arythmie. Si l'arythmie s'est arrêtée, l'appareil annule la délivrance du choc électrique de défibrillation et décharge ses condensateurs sans provoquer un choc électrique au cœur. Si l'arythmie persiste, il délivre une décharge électrique de haute tension, par exemple de 800 Volts, entre la bobine de la sonde de défibrillation SVD' et le boitier métallique. Après le traitement, le défibrillateur vérifie à nouveau le rythme. En cas d'échec, plusieurs chocs de défibrillation peuvent être délivrés à la suite.

La longévité du défibrillateur D dépend de l'autonomie de la réserve d'énergie embarquée et de la durée de vie de la sonde de défibrillation connectée au ventricule droit. Cette sonde de défibrillation présente un risque plus important de rupture en raison d'une fragilité, en comparaison avec qu'une sonde dédiée à la stimulation, telles que les sondes d'un stimulateur de type VR, DR ou CRT. Une infection propagée aux sondes à l'intérieur du cœur (endocardite infectieuse sur sonde) est grevée d'une très forte mortalité.

De plus, la sonde de défibrillation peut être abimée en raison des contraintes mécaniques qu'elle subit suite à la délivrance d'un choc électrique. Dans ce cas, une intervention chirurgicale est requise pour changer la sonde de défibrillation, ce qui augmente le risque d'infections. Si l'explantation des sondes de stimulation peut être difficile, celle des sondes de défibrillation est très compliquée en raison des adhérences aux tissus veineux développées au niveau des électrodes de type bobines.

La durée de vie des sondes de défibrillation est brève comparée à celle des sondes de stimulation cardiaque. Même si de récents progrès ont amélioré leur fiabilité, il arrive en pratique d'avoir à implanter deux, voire trois sondes successives de défibrillation à un patient au cours de son suivi avec des risques, toujours augmentés, d'infection du système.

Les défibrillateurs transveineux présentent des risques de rupture de l'isolant électrique entourant la sonde de défibrillation, rupture du conducteur métallique avec une perte de la fonction de stimulation, et perception de potentiels parasites électriques pouvant être interprétés à tort comme une arythmie ventriculaire et résultant en un choc électrique inapproprié.

La longévité des défibrillateurs est plus courte que celle d'un stimulateur cardiaque. Cela est surtout vrai pour les défibrillateurs triple chambres, très consommateurs en énergie.

Pour éviter les sondes endocavitaires de défibrillation, des défibrillateurs sous cutané de type S-ICD (pour « Subcutaneous Implantable Cardioverter Defibrillator » en anglais) ont été développés par Boston Scientific (marque déposée). La **figure 9** illustre un tel défibrillateur S-ICD. Le défibrillateur S-ICD comporte un boîtier métallique placé de façon postérieure sur la paroi latérale thoracique gauche, à une dizaine de centimètres en dessous du creux de l'aisselle, ce boitier étant relié à une sonde positionnée sous la peau, le long du bord gauche du sternum. La sonde est équipée de deux électrodes de détection et d'une bobine de défibrillation.

Contrairement à un défibrillateur transveineux, la sonde du défibrillateur sous-cutané S-ICD n'est pas connectée à une cavité du cœur. La détection du rythme ne se fait pas en analysant l'activité électrique directement au contact du muscle cardiaque. Les deux électrodes de détection sur la sonde et le boitier métallique dessinent un triangle avec trois axes électriques qui permettent de reconstituer un électrocardiogramme comparable à celui décrit en référence à la figure 1.

N'ayant pas d'électrode au contact direct du cœur, le défibrillateur sous-cutané ne possède pas de fonction de stimulation cardiaque, telle qu'une stimulation de type VR, DR, CTR ou ATP.

De plus, la détection de pathologies par le défibrillateur sous-cutané se fait à partir d'un électrocardiogramme reconstitué entre les électrodes portées par la sonde et le boitier. Cette détection est moins fiable qu'avec un défibrillateur transveineux. En effet, le défibrillateur sous-cutané ne comporte pas de sonde endocavitaire au contact direct du myocarde. La sensibilité de la détection dépend de la qualité du signal électrique détecté sur l'électrocardiogramme sous-cutané. Une faible amplitude du signal engendre un risque de sous détection et de non traitement d'une arythmie.

Le défibrillateur sous-cutané peut détecter par erreur une fausse arythmie, lorsque la personne qui porte le défibrillateur effectue un effort physique, ou en cas d'évolution de la maladie, ou en présence d'un bloc de branche droit BBD (dépolarisation tardive du ventricule droit) ou un bloc de branche gauche BBG (dépolarisation tardive du ventricule gauche). Le défibrillateur sous-cutané peut détecter des fréquences de signaux d'origine musculaire (appelées des myopotentiels) en cas de présence d'air autour de la sonde ou du boitier, à cause d'un effort physique ou en situation post opératoire. La spécificité de la détection d'une arythmie dépend de la qualité du signal cardiaque détecté en position sous cutanée avec un risque de surdétection de l'onde T de repolarisation sur l'ECG sous cutané, entrainant un risque de diagnostic par excès. Dans ces cas, le défibrillateur sous-cutané peut générer un choc électrique inapproprié.

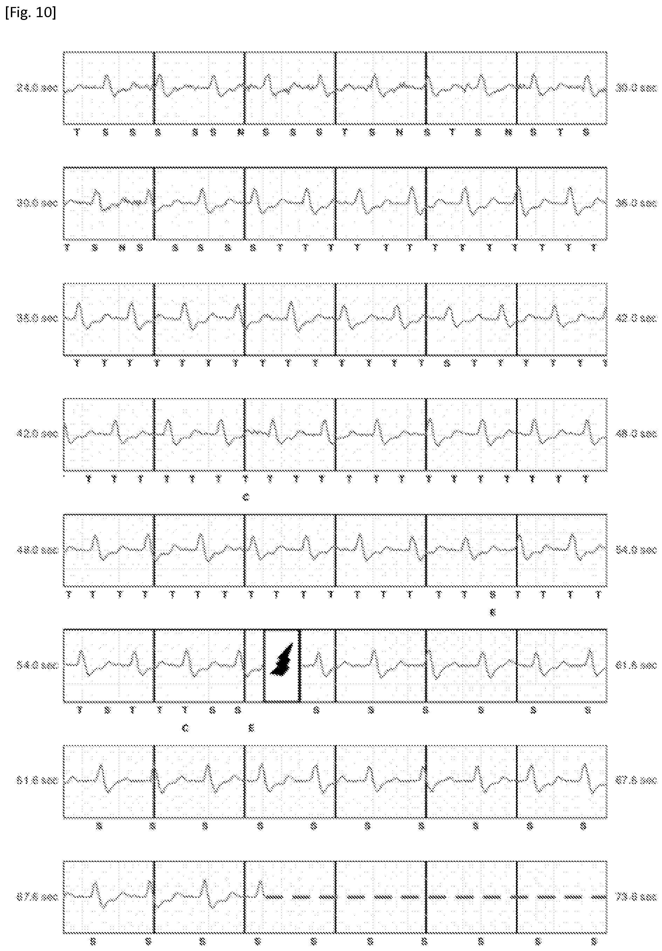

A titre indicatif, la **figure 10** présente un électrocardiogramme d'une personne ayant un stimulateur de type S-ICD implanté, qui était en train de courir. Les marqueurs S sur la première ligne signalent une activité cardiaque normale et correspondent à des ondes R. Les marqueurs T correspondent à un comptage inapproprié des ondes T de la repolarisation ventriculaire. Il s'agit donc d'une sur-détection. L'intervalle entre les ondes R et T étant court, le défibrillateur classe le rythme du cœur en tachycardie. Les intervalles courts sont de plus en plus fréquents avec l'accélération de la fréquence à l'effort, jusqu'à devenir permanents à la quatrième ligne. Le défibrillateur pause alors par erreur le diagnostic de fibrillation ventriculaire et charge ses condensateurs. A la sixième ligne, l'éclair désigne la délivrance d'un choc électrique inapproprié. L'effort s'arrête alors immédiatement et l'électrocardiogramme reprend une forme normale avec des marqueurs S à chaque battement cardiaque.

Les défibrillateurs S-ICD peuvent confondre différents types de tachycardie, par exemple entre une tachycardie ventriculaire, une tachycardie auriculaire et/ou une tachycardie supra-ventriculaire. De plus, les tests de détection réalisés dans la période préopératoire pour s'assurer du l'exploitation de l'ECG sous cutané avant d'implanter un défibrillateur sous-cutané ne donnent qu'une information ponctuelle qui peut être prise en défaut par l'évolution ultérieure, par exemple une apparition d'un bloc de branche droit ou gauche sur l'ECG qui modifie les conditions de détection, ou une variabilité du signal en fonction de la position ou de l'activité du patient.

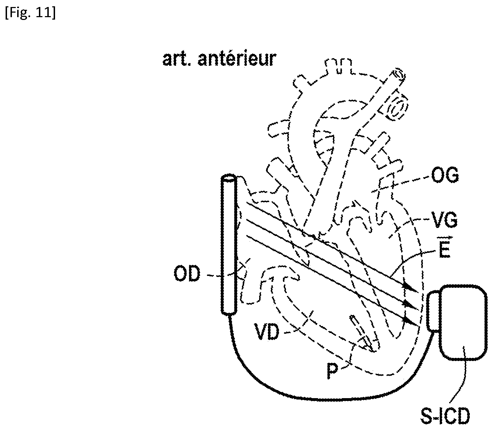

L'entreprise Boston Scientific (marque déposée) développe un système hybride qui peut effectuer une stimulation et/ou une défibrillation. Ce système, encore en phase d'étude, est publié dans l'article de Tjong et al., « Acute and 3-Month Performance of a Communicating Leadless ATP and S-ICD », JACC Clinical Electrophysiology, 2017. Ce système, illustré par la **figure 11****,** comporte un défibrillateur sous-cutané et un stimulateur de type capsule endo-ventricule droit, commandée par le défibrillateur, le défibrillateur et le stimulateur pouvant communiquer entre eux via un canal radio. Lorsque le défibrillateur détecte une arythmie, il peut commander en premier lieu le stimulateur pour effectuer une stimulation cardiaque, et en fonction du résultat de la stimulation, le défibrillateur peut émettre ou pas un choc électrique de défibrillation. Les documents US 2018/0056079, US 10238883 B2 et D5 US 9511233 B2 décrivent des systèmes similaires à celui de la figure 11, ces systèmes comportant un défibrillateur sous-cutané et des stimulateurs de type capsule.

Cette solution n'est pas satisfaisante, à cause des inconvénients des stimulateurs de type capsule décrits précédemment. De plus, cette solution de l'art antérieur repose sur des commandes générées par le défibrillateur sous cutané, alors que la fiabilité de la détection des arythmies et le diagnostic des pathologies par ce défibrillateur restent discutables.

Il existe donc un besoin en une solution permettant de traiter le plus possible de pathologies cardiaques, par une stimulation et/ou une défibrillation, et qui ne présente pas les inconvénients des méthodes connues de l'état de l'art, notamment la méthode de défibrillateur avec sonde endocavitaire illustré par la figure 7, et la méthode du système de Boston Scientific illustré par la figure 11.

### Exposé

Le présent enseignement vise un procédé de stimulation cardiaque, mis en œuvre par un stimulateur cardiaque implanté dans un corps d'un individu, le stimulateur comportant au moins une sonde dédiée pour la stimulation cardiaque, le procédé comportant :
- une étape d'écoute de l'activité électrique du cœur de l'individu via ladite au moins une sonde ;
- sur détection d'une arythmie cardiaque, une étape de stimulation cardiaque en fonction de l'arythmie cardiaque détectée via ladite au moins une sonde; et
- sur détection de déficience de la stimulation cardiaque, une étape d'envoi d'une commande de défibrillation cardiaque interprétable par un défibrillateur sous cutané implanté dans le corps dont toutes les sondes sont sous-cutanées.

Corrélativement, le présent enseignement vise un stimulateur cardiaque pouvant être implanté dans un corps d'un individu et comportant :
- au moins une sonde dédiée à la stimulation cardiaque;
- un module d'écoute configuré pour écouter l'activité électrique du cœur de l'individu via ladite au moins une sonde, le module d'écoute est configuré en outre pour détecter une arythmie cardiaque ;
- un module de stimulation configuré pour effectuer, sur détection d'une arythmie cardiaque, une stimulation cardiaque via ladite au moins une sonde en fonction de l'arythmie cardiaque détectée ; et
- un module de commande configuré pour envoyer, sur détection de déficience de la stimulation cardiaque, une commande de défibrillation cardiaque interprétable par un défibrillateur sous cutané, dont toutes les sondes sont sous-cutanées, pouvant être implanté dans le corps de l'individu.

Le stimulateur cardiaque selon le présent enseignement met en œuvre le procédé de stimulation cardiaque conforme au présent enseignement.

Les caractéristiques et avantages du procédé de stimulation cardiaque selon le présent enseignement et présentés ci-après s'appliquent de la même façon à un stimulateur cardiaque conforme au présent enseignement et vice-versa.

Le stimulateur conforme au présent enseignement comporte :
- un boitier comportant les modules d'écoute, de stimulation, et de commande, et une source d'alimentation électrique ; et
- la ou les sondes dédiées pour la stimulation cardiaque, ces sondes pouvant être connectées au ventricule droit, au ventricule gauche et/ou à l'oreillette droite.

Le stimulateur cardiaque conforme à l'invention peut être implanté dans la région de l'épaule gauche ou droite de l'individu, comme les stimulateurs de l'état de la technique illustrés par les figures 5A, 5B et 5C.

Le stimulateur cardiaque conforme au présent enseignement met en œuvre l'étape d'écoute de l'activité électrique du cœur de façon permanente.

A titre d'exemples, l'arythmie cardiaque détectée peut être une bradycardie. L'étape de stimulation cardiaque prend en considération le type de la bradycardie (née dans une oreillette ou dans un ventricule), ainsi, la stimulation peut être en mode VR, DR ou CRT. Le stimulateur conforme à l'invention peut effectuer une stimulation de type ATP, notamment lorsque l'arythmie détectée est de type tachycardie ventriculaire.

Conformément au présent enseignement, le stimulateur cardiaque ne comporte pas de sonde de défibrillation, toutes ses sondes étant dédiées à a stimulation cardiaque. Nous rappelons qu'une sonde dédiée à la stimulation est moins fragile, présente moins de risque d'infection, et peut être explantée plus facilement qu'une sonde de défibrillation telle que la sonde SVD' du défibrillateur avec sonde de la figure 7. Le stimulateur n'assure pas de fonction de défibrillation cardiaque. En cas d'une arythmie relative à une pathologie nécessitant une défibrillation, telle qu'une fibrillation ventriculaire, le stimulateur envoie la commande interprétable par le défibrillateur, qui effectuera la défibrillation cardiaque.

Le stimulateur conforme au présent enseignement peut comporter une seule sonde, par exemple une sonde connectée au muscle du ventricule droit. Alternativement, le stimulateur conforme à l'invention peut comporter plusieurs sondes dont chacune est connectée à une cavité du cœur, en priorité le ventricule droit, le ventricule gauche, l'oreillette droite puis l'oreillette gauche.

Contrairement aux stimulateurs endocavitaires (les capsules) de l'art antérieur illustrés par les figures 6 et 9, le stimulateur conforme à l'invention peut comporter plusieurs sondes dédiées à la stimulation et peut donc effectuer plusieurs types de stimulation en fonction du nombre et de l'emplacement de ses sondes, les capsules de l'art antérieur ne pouvant stimuler que le ventricule droit.

Même en supposant qu'il y aurait des stimulateurs endocavitaires de type capsules implantables dans les oreillettes ou dans le ventricule gauche, une stimulation de différentes cavités du cœur nécessite une implantation de plusieurs stimulateurs endocavitaires, chacun dans une cavité (oreillette et/ou ventricule), et une communication entre les différents stimulateurs pour coopérer et synchroniser leurs fonctionnements. Une telle solution aurait un coût élevé et une technique d'implantation complexe. De plus, dans l'hypothèse d'une stimulation double chambre ou triple chambre, la communication entre les différents stimulateurs consomme de l'énergie. Enfin, le nombre de stimulateurs implantés dans le cœur peut augmenter à terme, en cas de défaillance de certains stimulateurs.

Le stimulateur conforme au présent enseignement ne présente pas ces inconvénients car toutes les sondes, quel que soit leur nombre, sont connectées au même boitier comportant les modules d'écoute et de stimulation.

Contrairement aux stimulateurs de type capsule, le stimulateur conforme au présent enseignement peut être retiré ou remplacé facilement. En particulier, le stimulateur conforme au présent enseignement peut être remplacé complètement ou partiellement, par exemple en remplacement une sonde ou le boitier uniquement, ou en rajoutant une sonde supplémentaire si nécessaire.

Le stimulateur conforme au présent enseignement est plus avantageux que les stimulateurs de l'art antérieur illustrés par les figures 5A à 5C, car il a des moyens pour envoyer la commande de défibrillation. En association avec le défibrillateur, il peut traiter plus de pathologies cardiaques.

Le procédé de stimulation conforme au présent enseignement permet d'essayer en premier lieu de traiter l'arythmie détectée par la stimulation cardiaque. Seulement lorsque la stimulation ne suffit pas pour traiter cette arythmie, le stimulateur conforme au présent enseignement envoie la commande de défibrillation.

La déficience de la stimulation peut être détectée lorsque l'arythmie cardiaque détectée persiste après la mise en œuvre de l'étape de stimulation, ou lorsqu'une nouvelle arythmie cardiaque est détectée pendant une durée déterminée comptée à partir de la mise en œuvre de l'étape de stimulation. A titre d'exemple, la déficience de la stimulation cardiaque peut être détectée suite à une détection d'une fibrillation cardiaque.

Le stimulateur cardiaque conforme au présent enseignement peut délivrer une stimulation de type ATP en cas de détection d'une arythmie de type tachycardie ventriculaire. Nous notons qu'en pratique, une stimulation ATP est susceptible de transformer la tachycardie ventriculaire en fibrillation ventriculaire, nécessitant alors un choc de défibrillation. Pour cette raison, sur avis d'un médecin, lorsque le stimulateur cardiaque conforme au présent enseignement est configuré en outre pour effectuer des stimulations ATP, il peut être utilisé en association avec un défibrillateur conforme au présent enseignement, décrit en ce qui suit.

Le présent enseignement vise également un procédé de défibrillation cardiaque, mis en œuvre par un défibrillateur sous cutané implanté dans un corps d'un individu et comportant des condensateurs et des électrodes de défibrillation, toutes les sondes du défibrillateur sont sous-cutanées, ce procédé comporte :
- une étape d'obtention d'une commande de défibrillation générée par un stimulateur cardiaque implanté dans le corps de l'individu ; et
- sur obtention de ladite commande, une étape de défibrillation cardiaque en fonction de la commande obtenue par une décharge des condensateurs dans les électrodes de défibrillation.

Corrélativement, le présent enseignement vise un défibrillateur cardiaque sous cutané pouvant être implanté dans un corps d'un individu et comportant :
- au moins une sonde comportant une électrode de défibrillation, toutes les sondes du défibrillateur sous-cutané sont sous-cutanées ; et
- un boitier constituant une électrode de défibrillation et comportant :
   - des condensateurs ;
   - un module d'obtention d'une commande configuré pour obtenir une commande de défibrillation générée par un stimulateur cardiaque pouvant être implanté également dans le corps de l'individu ; et
   - un module de défibrillation configuré pour effectuer, sur obtention de ladite commande, une défibrillation cardiaque en fonction de la commande obtenue par une décharge des condensateurs dans les électrodes de défibrillation.

Le défibrillateur cardiaque conforme au présent enseignement met en œuvre le procédé de défibrillation cardiaque conforme au présent enseignement.

Les caractéristiques et avantages du procédé de défibrillation cardiaque selon le présent enseignement présentés ci-après s'appliquent de la même façon à un défibrillateur cardiaque conforme au présent enseignement, et vice-versa.

Le défibrillateur cardiaque conforme au présent enseignement est implanté dans le corps comme un défibrillateur de type S-ICD de l'état de la technique, tel que celui illustré par la figure 9. Le défibrillateur conforme au présent enseignement comporte :
- un boitier implanté sur la paroi latérale thoracique gauche ou droite, comportant le module d'obtention d'une commande, le module de défibrillation, les condensateurs et une source d'alimentation électrique ; et
- au moins une sonde de défibrillation, comportant lesdites électrodes, positionnée sous la peau au long du bord du sternum ; les électrodes comportent typiquement deux électrodes de détection et une électrode de défibrillation de type bobine par exemple.

Le procédé de défibrillation conforme au présent enseignement permet d'éviter des défibrillations inappropriées. En effet, lorsque le défibrillateur est implanté en association avec le stimulateur, la défibrillation n'est mise en œuvre que sur obtention de la commande générée par le stimulateur, le stimulateur pouvant détecter les arythmies cardiaques et diagnostiquer les pathologies avec plus de fiabilité vu qu'il a une connexion directe avec au moins une cavité du cœur.

Le fait d'éviter les défibrillations et les chocs électriques inappropriés permet d'augmenter la durée de vie du défibrillateur, et ainsi de réduire le nombre d'interventions éventuellement requises pour la maintenance du défibrillateur. Nous rappelons que le défibrillateur est implanté dans le corps de l'individu et que chaque intervention peut nécessiter une opération chirurgicale et présente un risque pour la santé de l'individu.

Contrairement aux défibrillateurs trans-veineux comme celui décrit en référence à la figure 7, le défibrillateur conforme au présent enseignement ne comporte pas de sonde endocavitaire et présente donc moins de risque d'infections ou de complications dues à la présence d'une sonde de défibrillation endocavitaire. Toutes les sondes du défibrillateur sous-cutané selon le présent enseignement sont sous-cutanées, situées sous la peau (région sous-cutanée) à l'extérieur du cœur.

En comparaison avec les défibrillateurs trans-veineux, l'implantation et les éventuelles interventions de maintenance du défibrillateur conforme à l'invention comporte moins de risque.

Le défibrillateur conforme au présent enseignement permet d'obtenir un bon compromis entre la réduction du risque de chocs électriques inappropriés, et la réduction du risque de complications liées aux sondes de défibrillation endocavitaires.

Le présent enseignement vise également un procédé de traitement d'au moins un problème cardiaque, ce procédé étant mis en œuvre par un système comportant un stimulateur cardiaque et un défibrillateur sous cutané implantés dans un corps d'un individu. Le procédé de traitement comporte les étapes d'un procédé de stimulation conforme au présent enseignement et les étapes d'un procédé de défibrillation conforme au présent enseignement.

Corrélativement, le présent enseignement vise un système de traitement d'au moins un problème cardiaque comportant un stimulateur cardiaque et un défibrillateur sous cutané, le stimulateur et le défibrillateur étant conformes au présent enseignement.

Les caractéristiques et avantages du procédé de stimulation et du procédé de défibrillation selon le présent enseignement déjà présentés s'appliquent de la même façon au procédé de traitement d'au moins un problème cardiaque conforme au présent enseignement, et vice-versa.

Plusieurs pathologies du cœur peuvent être traitées par le système conforme au présent enseignement. A titre d'exemple, une bradycardie peut être traitée par une stimulation mise en œuvre par le stimulateur conforme au présent enseignement. Une désynchronisation interventriculaire peut être traitée par un stimulateur cardiaque conforme au présent enseignement, triple chambre CRT. Une tachycardie ventriculaire peut être traitée par une stimulation ATP délivrée par le stimulateur cardiaque, suivie éventuellement par un choc électrique de défibrillation délivré par le défibrillateur, les condensateurs du défibrillateur pouvant être chargés en parallèle avec la stimulation ATP. Une fibrillation ventriculaire peut être traitée par une première salve de stimulation ATP délivrée par le stimulateur cardiaque pendant la charge du défibrillateur, éventuellement suivie par un choc électrique de défibrillation délivré par le défibrillateur conforme au présent enseignement.

Dans la solution de l'art antérieur décrite en référence à la figure 11, un défibrillateur sous-cutané commande un stimulateur. Au contraire, dans le présent enseignement, c'est le stimulateur qui commande le défibrillateur en envoyant la commande interprétable par le défibrillateur. Cette caractéristique permet une meilleure fiabilité pour détecter les arythmies cardiaques et pour éviter les défibrillations inappropriées, car c'est le stimulateur, comportant une sonde endocavitaire, qui prends sa décision pour la stimulation et qui envoie la commande de défibrillation.

Dans un mode de réalisation, le procédé de défibrillation cardiaque comporte en outre:
- une étape d'écoute de l'activité électrique du cœur de l'individu; et
- une étape de détection d'une arythmie cardiaque ;
l'étape de défibrillation prenant en outre en compte l'arythmie cardiaque détectée.

Dans ce mode, le défibrillateur conforme au présent enseignement comporte en outre un module d'écoute de l'activité électrique du cœur de l'individu, ce module comportant des électrodes d'écoute et étant configuré pour écouter l'activité électrique du cœur et pour détecter une arythmie cardiaque, le module de défibrillation étant configuré pour effectuer la défibrillation en prenant en outre en compte l'arythmie cardiaque détectée, ledit boitier constituant une dite électrode d'écoute, ladite au moins une sonde comportant au moins une dite électrode d'écoute.

Dans ce mode, le défibrillateur met en œuvre l'étape d'écoute de l'activité électrique du cœur de façon permanente.

Ce mode permet une double surveillance de l'activité électrique du cœur, par le stimulateur et par le défibrillateur.

L'écoute de l'activité électrique du cœur par le défibrillateur lui permet de préparer une éventuelle intervention en cas de détection d'une arythmie et d'intervenir plus rapidement. En effet, le défibrillateur peut analyser l'arythmie cardiaque détectée, déterminer la pathologie relative à cette arythmie et en déduire le type de défibrillation qu'il doit effectuer lorsqu'il obtient une commande générée par le stimulateur, par exemple la puissance du choc électrique, la polarité du choc électrique, le mode de délivrance du choc électrique, etc. Ainsi, si le défibrillateur obtient la commande de défibrillation, il peut réagir rapidement vu qu'il a déjà déterminé le type de défibrillation à effectuer.

Lorsque le défibrillateur obtient une commande générée par le stimulateur, cette commande peut confirmer la justesse de l'analyse de l'arythmie détectée par le défibrillateur, comme elle peut constituer une correction de celle-ci. Le défibrillateur conforme au présent enseignement prend compte de la commande et de sa détection de l'arythmie. Toutefois, la commande obtenue est prise en compte avec priorité car le stimulateur détecte mieux les arythmies cardiaques grâce à sa ou ses sondes endocavitaires. A titre d'exemple, lorsque le stimulateur détermine qu'une arythmie détectée est une tachycardie auriculaire, le stimulateur n'envoie pas de commande au défibrillateur et le défibrillateur ne génère pas de choc électrique même si le défibrillateur a considéré par erreur que l'arythmie est une tachycardie ventriculaire. Le stimulateur permet d'ajuster le diagnostic effectué par le défibrillateur.

Le stimulateur conforme au présent enseignement peut faire la différence entre les différents types de tachycardie en exécutant un algorithme diagnostic de l'art antérieur. Par exemple, le stimulateur peut déterminer la cavité du cœur à l'origine de la tachycardie, grâce à ses sondes endocavitaires. Selon un autre exemple, le stimulateur conforme à l'invention peut dissocier les rythmes, calculer et comparer les fréquences électriques des oreillettes et des ventricules. Si la fréquence au niveau des ventricules est plus rapide que celle au niveau des oreillettes, le stimulateur détermine que l'arythmie est une tachycardie ventriculaire.

Le défibrillateur peut connaître la pathologie correspondant à l'arythmie détectée en exécutant un ou plusieurs algorithmes diagnostiques programmés pour identifier la pathologie correspondante à l'arythmie détectée.

Dans un mode de réalisation, le procédé de défibrillation selon le présent enseignement comporte en outre une étape de recharge des condensateurs sur obtention de la commande de défibrillation. Même s'il détecte une arythmie cardiaque, le défibrillateur attend qu'il obtienne une commande générée par le stimulateur pour charger ses condensateurs. En effet, le défibrillateur peut détecter une arythmie par erreur, il attend alors la commande du stimulateur pour éviter que les condensateurs soient chargées pour rien.

Dans un autre mode de réalisation, lorsque l'arythmie cardiaque détectée par le défibrillateur conforme au présent enseignement est une fibrillation ventriculaire, le procédé de défibrillation comporte en outre, sur détection de ladite arythmie cardiaque:
- une étape de déclenchement d'un compte à rebours temporel pour une durée d'attente déterminée ;
- une étape de recharge des condensateurs ; et
- en absence d'obtention d'une commande à l'expiration de la durée d'attente, une étape de décharge des condensateurs dans le corps,
l'étape de défibrillation cardiaque n'étant mise en œuvre que sur obtention d'une commande avant l'expiration de la durée d'attente.

Dans ce mode de réalisation, le défibrillateur cardiaque sous cutané conforme au présent enseignement comporte en outre un module de déclenchement d'un compte à rebours temporel pour une durée d'attente déterminée, sur détection d'une arythmie cardiaque de type fibrillation ventriculaire. Lorsque l'arythmie cardiaque détectée est une fibrillation ventriculaire, le module de défibrillation est configuré pour :
- charger les condensateurs ;
- décharger les condensateurs dans le corps en absence d'obtention d'une commande à l'expiration de la durée d'attente; et
- n'effectuer la défibrillation cardiaque que sur obtention d'une commande avant l'expiration de la durée d'attente.

En effet, une arythmie cardiaque de type fibrillation ventriculaire présente un risque vital qu'il faut traiter en urgence dans un délai maximum de deux minutes. Ce mode permet de gagner le temps nécessaire pour la recharge des condensateurs, au cas où le défibrillateur obtient une commande de défibrillation.

Si le défibrillateur a détecté par erreur une arythmie de type fibrillation ventriculaire, il n'obtient pas de commande de défibrillation. Le défibrillateur décharge ses condensateurs dans le corps de l'individu après l'expiration de la durée d'attente sans provoquer de choc électrique. Dans ce cas, la décharge des condensateurs se fait lentement et progressivement de façon à ne pas causer un danger pour la santé de l'individu.

Dans un mode de réalisation, le défibrillateur conforme au présent enseignement comporte deux sondes, dont chacune comporte une électrode de détection de l'arythmie cardiaque, et une électrode dédiée à la défibrillation. La détection de l'arythmie cardiaque se fait en analysant un électrocardiogramme enregistré sur trois vecteurs : un vecteur reconstruit entre les deux électrodes de détection, deux vecteurs reconstruits chacun entre une desdites électrodes de détection et le boitier. Les électrodes de défibrillation sont branchées en parallèles et connectées à un pôle unique de défibrillation à l'intérieur d'un bloc connecteur entre le boitier et les deux sondes. Le choc de défibrillation est délivré de façon simultanée à partir des deux électrodes de défibrillation en direction du boitier (sens direct) ou à partir du boitier en direction des deux électrodes de défibrillation (sens inversé).

Dans un mode de réalisation, selon le procédé de traitement d'au moins un problème cardiaque, le stimulateur envoie la commande au défibrillateur via un canal de communication radio ou autre moyen de communication.

Dans un autre mode de réalisation, dans le procédé de traitement d'au moins un problème cardiaque selon le présent enseignement:
- l'étape d'envoi, par le stimulateur conforme au présent enseignement, d'une commande de défibrillation cardiaque comporte une propagation d'un signal électrique clef, d'une forme donnée, dans le corps de l'individu ; et
- l'étape d'obtention, par le défibrillateur conforme au présent enseignement, d'une commande de défibrillation comporte une détection du signal clef, le défibrillateur mettant en œuvre l'étape d'écoute de l'activité électrique du cœur.

Dans ce mode, le stimulateur et le défibrillateur sont configurés au préalable par rapport à la mise en œuvre du procédé de traitement, pour assurer une cohérence de l'interprétation du signal clef. Ce mode ne requiert pas une communication directe entre le stimulateur et le défibrillateur et ne présente donc pas de contrainte de synchronisation. Le défibrillateur détecte le signal clef vu qu'il met en œuvre l'étape d'écoute de façon permanente.

Conformément à ce mode de réalisation, le signal clef a une faible tension, de l'ordre de 1 volt, émis par exemple par l'anode d'une sonde ventriculaire droite du stimulateur conforme au présent enseignement.

Le signal étant anodique et de faible amplitude, le risque de stimulation du ventricule est écarté. L'amplitude étant supérieure à celle des signaux électriques musculaires (moins de 30 mVolt), le risque que le signal clef soit masqué par l'activité cardiaque est aussi écarté. Le signal clé peut être un signal électrique unipolaire émis par l'anode de la sonde ventriculaire droite du stimulateur cardiaque conforme au présent enseignement.

Aucune limitation n'est imposée sur la forme du signal clef, elle peut sinusoïdale, carrée ou triangulaire par exemple.

Le stimulateur conforme au présent enseignement peut être configuré pour ne pas effectuer de stimulation ATP lorsqu'il est implanté seul dans le corps de l'individu, par exemple en cas de retrait du défibrillateur de façon définitive, ou pour le remplacement ou la maintenance du défibrillateur.

Le défibrillateur conforme au présent enseignement peut comporter un module de sélection de mode pour mettre en œuvre ou pas l'écoute de l'activité électrique du cœur. Ce module est configuré pour sélectionner le mode où l'écoute est mise en œuvre lorsque le défibrillateur est implanté seul, par exemple en absence d'indication de stimulation cardiaque ou en cas de retrait du stimulateur, que soit définitivement ou pour une maintenance.

La technique proposée permet, chez un patient porteur d'un défibrillateur sous cutané conforme au présent enseignement, de:
- pouvoir associer un stimulateur conforme au présent enseignement permettant de délivrer une stimulation cardiaque selon un mode adapté aux besoins du patient avec une, deux ou trois sondes de stimulation selon les cas, le stimulateur pouvant être implanté après l'implantation du défibrillateur, par exemple après plusieurs années si l'évolution de la maladie le rend nécessaire ;
- pouvoir faire évoluer le système au cours de la vie du patient, par exemple en ajoutant des sondes de stimulation ou en modifiant une programmation du stimulateur de façon à ne pas utiliser une sonde devenue inutile;
- augmenter la sensibilité et la spécificité de la détection des arythmies cardiaques en recueillant directement le signal au contact du muscle cardiaque par la ou les sondes endocavitaires, par exemple placées dans le ventricule et l'oreillette droite ; et
- assurer une plus grande sécurité du système conforme au présent enseignement en laissant la commande au stimulateur, c'est-à-dire à celui des deux appareils qui a la plus grande sensibilité et la meilleure spécificité de détection des arythmies.

Le présent enseignement vise également un premier programme d'ordinateur sur un support d'enregistrement, ce programme étant susceptible d'être mis en œuvre dans un ordinateur ou un stimulateur conforme au présent enseignement. Ce programme comporte des instructions adaptées à la mise en œuvre d'un procédé de stimulation cardiaque tel que décrit ci-dessus.

Le présent enseignement vise également un deuxième programme d'ordinateur sur un support d'enregistrement, ce programme étant susceptible d'être mis en œuvre dans un ordinateur ou un défibrillateur conforme au présent enseignement. Ce programme comporte des instructions adaptées à la mise en œuvre d'un procédé de défibrillation cardiaque tel que décrit ci-dessus.

Le présent enseignement vise également un troisième programme d'ordinateur sur un support d'enregistrement, ce programme étant susceptible d'être mis en œuvre dans un ordinateur ou un système, conforme au présent enseignement, de traitement d'au moins un problème cardiaque. Ce programme comporte des instructions adaptées à la mise en œuvre d'un procédé de traitement d'au moins un problème cardiaque tel que décrit ci-dessus. En particulier, le troisième programme peut être construit à partir du premier et du deuxième programme.

Chacun de ces programmes peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

Le présent enseignement vise aussi un support d'information ou un support d'enregistrement lisibles par un ordinateur, et comportant des instructions du premier, du deuxième ou du troisième programme d'ordinateur tel que mentionné ci-dessus.

Les supports d'information ou d'enregistrement peuvent être n'importe quelle entité ou dispositif capable de stocker les programmes. Par exemple, les supports peuvent comporter un moyen de stockage, tel qu'une ROM, par exemple un CD ROM ou une ROM de circuit microélectronique, ou encore un moyen d'enregistrement magnétique, par exemple une disquette (floppy disc) ou un disque dur, ou une mémoire flash.

D'autre part, les supports d'information ou d'enregistrement peuvent être des supports transmissibles tels qu'un signal électrique ou optique, qui peut être acheminé via un câble électrique ou optique, par lien radio, par lien optique sans fil ou par d'autres moyens.

Les programmes selon le présent enseignement peuvent être en particulier téléchargés sur un réseau de type Internet.

Alternativement, chaque support d'informations ou d'enregistrement peut être un circuit intégré dans lequel un programme est incorporé, le circuit étant adapté pour exécuter ou pour être utilisé dans l'exécution du procédé de stimulation cardiaque conforme au présent enseignement ou du procédé de défibrillation cardiaque conforme au présent enseignement, ou du procédé de traitement d'au moins un problème cardiaque conforme au présent enseignement.

### Brève description des dessins

D'autres caractéristiques et avantages de la présente divulgation ressortiront de la description faite ci-dessous, en référence aux dessins annexés qui en illustrent un exemple de réalisation dépourvu de tout caractère limitatif. Sur les figures :
[Fig. 1] la figure 1, déjà décrite, illustre une activité électrique normale d'un cœur humain ;
[Fig. 2A-2B] les figures 2A et 2B, déjà décrites, illustrent une activité électrique de cœurs atteints de bradycardies ;
[Fig. 3] la figure 3, déjà décrite, illustre une activité électrique d'un cœur atteint d'une tachycardie ventriculaire ;
[Fig. 4] la figure 4, déjà décrite, illustre une activité électrique d'un cœur atteint d'une fibrillation ventriculaire ;
[Fig. 5A-5C] les figures 5A, 5B et 5C, déjà décrites, illustrent des exemples de stimulateurs cardiaques de l'art antérieur, avec au moins une sonde ;
[Fig. 6] la figure 6, déjà décrite, illustre un stimulateur cardiaque de type capsule de l'art antérieur ;
[Fig. 7] la figure 7, déjà décrite, illustre un défibrillateur cardiaque transveineux de l'art antérieur ;
[Fig. 8] la figure 8, déjà décrite, illustre une tachycardie ventriculaire traitée par une stimulation ATP ;
[Fig. 9] la figure 9, déjà décrite, illustre un défibrillateur cardiaque de type S-ICD de l'art antérieur ;
[Fig. 10] la figure 10, déjà décrite, illustre un choc électrique inapproprié délivré par un défibrillateur S-ICD de l'art antérieur ;
[Fig. 11] la figure 11, déjà décrite, illustre un système de l'art antérieur, comportant un stimulateur cardiaque de type capsule et un défibrillateur cardiaque de type S-ICD ;
[Fig. 12] la figure 12 illustre un système de traitement d'un problème cardiaque, un stimulateur cardiaque et un défibrillateur cardiaque conformes au présent enseignement;
[Fig. 13] la figure 13 est un organigramme représentant des étapes des procédés conformes au présent enseignement, mis en œuvre selon un mode de réalisation ;
[Fig. 14] la figure 14 présente des architectures fonctionnelles d'un système de traitement d'au moins un problème cardiaque, d'un stimulateur cardiaque et d'un défibrillateur cardiaque, conformes au présent enseignement;
[Fig. 15] la figure 15 illustre un défibrillateur cardiaque avec deux sondes, conforme au présent enseignement;
[Fig. 16] la figure 16 illustre un connecteur de type IS1 utilisé pour les sondes du défibrillateur de la figure 15 ;
[Fig. 17] la figure 17 illustre une implantation possible du défibrillateur de la figure 15 ;
[Fig. 18] la figure 18 présente une architecture matérielle d'un stimulateur cardiaque selon un mode de réalisation ; et
[Fig. 19] la figure 19 présente une architecture matérielle d'un défibrillateur cardiaque selon un mode de réalisation.

### Description des modes de réalisation

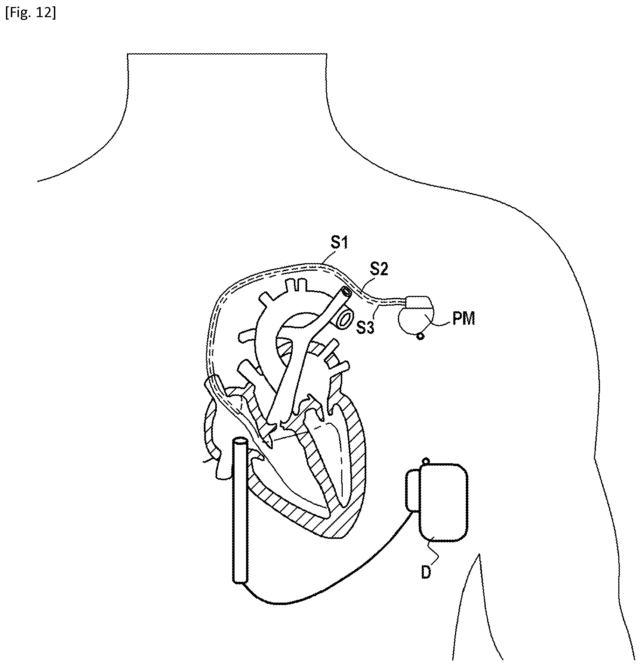

La **figure 12** illustre un système SYS, conforme au présent enseignement, de traitement d'au moins un problème cardiaque, selon un mode de réalisation.

Ce système SYS comporte un stimulateur PM et un défibrillateur D, tous les deux conformes à au présent enseignement. Le système SYS peut traiter des pathologies du cœur qui nécessitent une stimulation cardiaque et/ou une défibrillation cardiaque.

Le stimulateur PM comporte au moins une sonde dédiée à la stimulation, de préférence connectée au ventricule droit. Dans le mode décrit ici, le stimulateur PM comporte trois sondes S1, S2 et S3 dédiées à la stimulation, dont S1 est connectée au muscle du ventricule droit, S2 est connectée au muscle du ventricule gauche et S3 est connectée à l'oreillette droite.

Dans un autre mode de réalisation, le stimulateur PM comporte en outre une quatrième sonde connectée à l'oreillette gauche.

Les trois sondes S1, S2 et S3 sont reliées à un boitier du stimulateur cardiaque, implanté dans le corps d'un individu, dans la région de l'épaule gauche. Via ses sondes, le stimulateur PM peut écouter, de façon permanente, l'activité électrique de chacune des trois cavités cardiaques (ventricule droit, ventricule gauche et oreillette droite), détecter des arythmies, et stimuler au moins une cavité du cœur, par exemple stimuler le ventricule droit par la sonde S1.

Le défibrillateur D est un défibrillateur sous-cutané, de type S-ICD, implanté dans le corps de l'individu. Le défibrillateur D comporte un boîtier métallique placé de façon postérieure sur la paroi latérale thoracique gauche, et relié à une sonde équipée d'électrodes positionnée sous la peau, le long du bord gauche du sternum, les électrodes comprenant deux électrodes de détection et une électrode de défibrillation comportant une bobine.

Le défibrillateur D comporte dans son boitier des condensateurs qu'il peut charger, puis les décharger pour délivrer un choc électrique entre l'électrode de défibrillation et le boitier. Le choc électrique traverse la masse musculaire du cœur et résulte en une défibrillation cardiaque.

Ni le stimulateur PM ni le défibrillateur D ne comporte une sonde endocavitaire de défibrillation cardiaque. Toutes les sondes du défibrillateur D sont sous-cutanées.

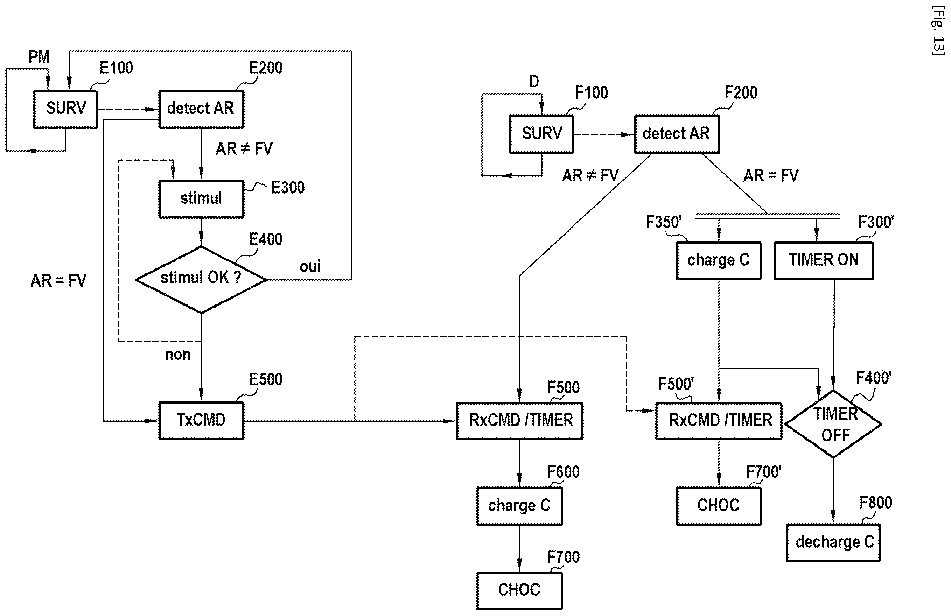

La **figure 13** est un organigramme représentant des étapes du procédé de traitement d'au moins un problème cardiaque mis en œuvre par le système SYS, selon un mode de réalisation. Le procédé de traitement d'au moins un problème cardiaque comporte :
- les étapes d'un procédé de stimulation conforme au présent enseignement, mis en œuvre par le stimulateur PM ; et
- les étapes d'un procédé de défibrillation conforme au présent enseignement, mis en œuvre par le défibrillateur D.

En particulier, les étapes E100, E200, E300, E400 et E500 décrites ci-après sont des étapes du procédé de stimulation le présent enseignement. Les étapes F100, F200, F400, F500, F600, F700, F300', F350', F400', F500', F700' et F800' décrites ci-après sont des étapes du procédé de défibrillation selon le présent enseignement.

Dans le mode décrit ici, le stimulateur PM et le défibrillateur D écoutent et surveillent tous les deux, de façon permanente, l'activité électrique du cœur, au cours de deux étapes E100 et F100 mises en œuvre en boucle, respectivement par le stimulateur PM et le défibrillateur D.

Nous supposons que le cœur de l'individu subit une pathologie et qu'il présente une arythmie dans son activité électrique.

Au cours d'une étape E200, le stimulateur PM détecte l'arythmie cardiaque. Le défibrillateur la détecte également au cours d'une étape F200. Nous rappelons que la détection de l'arythmie par le stimulateur est plus fiable car il a des sondes endocavitaires.

Au cours de l'étape E200, le stimulateur PM analyse l'arythmie cardiaque détectée, en exécutant des algorithmes diagnostiques programmés pour identifier la pathologie correspondante à l'arythmie détectée. Ces algorithmes déterminent la nature de l'arythmie en fonction plusieurs critères tels que la fréquence de l'arythmie, la régularité de l'activité électrique, un mode de démarrage (rapide ou progressif) de l'arythmie, une séquence de type A-V lorsque le stimulateur comporte une sonde S3 connectée à l'oreillette droite, l'origine du premier battement de l'arythmie (oreillette ou ventricule), la modification de la forme des complexes QRS, etc. Plus de critères considérés par les algorithmes diagnostiques, plus la détection est fiable.

En parallèle, le défibrillateur D analyse également, au cours de l'étape F200, l'arythmie cardiaque détectée. Nous supposons, dans un premier exemple, que l'arythmie cardiaque ne correspond pas à une fibrillation ventriculaire. Le défibrillateur D ne met en œuvre aucune étape autre que l'étape d'écoute F100.

Au cours d'une étape E300, le stimulateur PM stimule le cœur en fonction de l'arythmie cardiaque détectée. Par exemple, lorsque l'arythmie cardiaque correspond à une bradycardie, le stimulateur PM stimule le ventricule droit via la sonde S1, et éventuellement le ventricule gauche via la sonde S2, tout en synchronisant cette ou ces stimulations avec l'activité électrique de l'oreillette droite, écoutée via la sonde S3.

Lorsque l'arythmie cardiaque correspond à une tachycardie ventriculaire, le stimulateur PM effectue une stimulation de type ATP du ventricule droit via la sonde S1avec une fréquence supérieure à celle de la tachycardie détectée.

Le stimulateur continue en parallèle à écouter (E100) l'activité électrique du cœur.

Au cours d'une étape E400, le stimulateur PM vérifie si la simulation a réussi ou pas à traiter l'arythmie cardiaque détectée. L'étape E400 est mise en œuvre immédiatement après la mise en œuvre de la stimulation E300.

Si le stimulateur PM détecte au cours de l'étape E400 que la simulation est réussie, le stimulateur PM revient au mode de surveillance normale, en mettant en œuvre l'étape d'écoute E100.

Si au cours de l'étape E400, le stimulateur PM détecte une déficience de la stimulation E300, alors soit :
- il envoie directement, au cours d'une étape E500, une commande de défibrillation CMD interprétable par le défibrillateur D, soit
- il remet en œuvre les étapes E300 et E400 pour une ou plusieurs nouvelles salves de stimulation et une vérification de l'effet de la stimulation après chaque salve. La mise en œuvre des étapes E300 et E400 peut être répétée jusqu'à un nombre maximum de fois. Par exemple, lorsque l'arythmie détectée (E200) est une tachycardie ventriculaire avec un cycle de 320 ms, le stimulateur effectue en premier lieu une stimulation (E300) ATP avec un cycle de 300ms ; s'il vérifie (E400) une déficience de cette première stimulation, il effectue une deuxième stimulation (E300) ATP avec un cycle de 280 ms ; ensuite une troisième salve de stimulation avec un cycle de 260 ms en cas de déficience de la deuxième stimulation, et enfin, si même la troisième stimulation est déficiente, le stimulateur envoie au cours de l'étape E500 la commande de défibrillation CMD.

La déficience de la stimulation peut être détectée (E400) par une détection de la persistance de l'arythmie cardiaque détectée au cours de l'étape E200, même après la stimulation E300. Alternativement, la déficience de la stimulation peut être détectée (E400) par une détection d'une nouvelle arythmie cardiaque, telle qu'une fibrillation ventriculaire après une tachycardie ventriculaire.

Nous supposons dans cet exemple que le stimulateur PM a détecté (E400) une déficience de la stimulation et qu'il a envoyé (E500) la commande CMD.

Au cours d'une étape F500 du procédé de défibrillation conforme à l'invention, le défibrillateur D obtient la commande CMD.

Sur obtention de la commande CMD, le défibrillateur D charge ses condensateurs au cours d'une étape F600.

Au cours d'une étape F700, le défibrillateur effectue une défibrillation cardiaque en déchargeant les condensateurs dans l'électrode de défibrillation. Cette décharge cause un choc électrique entre les électrodes et le boitier du défibrillateur D, affectant le muscle du cœur.

Dans le mode décrit ici, la défibrillation F700 prend compte de la commande CMD, mais aussi de l'arythmie cardiaque détectée par le défibrillateur D au cours de l'étape F200.

Dans le premier exemple déjà décrit, l'arythmie détectée au cours de l'étape F200 ne correspond pas à une fibrillation ventriculaire.

Dans un deuxième exemple, nous supposons que le défibrillateur a détecté au cours de l'étape F200 une arythmie cardiaque correspondant à une fibrillation ventriculaire.

Comme il s'agit d'une pathologie à risque vital, suite à la détection F200, le défibrillateur charge ses condensateurs au cours d'une étape F350' et déclenche un compte à rebours temporel TIMER au cours d'une étape F300'. De préférence, les étapes F350' et F300 sont mises en œuvre simultanément ou à des instants sensiblement proches.

Pendant la durée du compte à rebours TIMER, le défibrillateur attend à ce qu'il obtienne une commande de défibrillation en provenance du stimulateur PM.

Selon une première hypothèse, nous supposons que le stimulateur PM détecte également au cours de l'étape E200 une arythmie de type fibrillation. Le stimulateur envoie (E500) la commande de défibrillation CMD, soit directement suite à la détection E200, soit après un nombre donné de stimulations (E300) et de vérifications (E400). Le défibrillateur D obtient, au cours d'une étape F500', une commande CMD de défibrillation générée par le stimulateur PM. Les condensateurs étant déjà chargés, sur obtention de la commande CMD, le défibrillateur D met en œuvre une étape de défibrillation cardiaque F700' en fonction de la commande obtenue CMD par une décharge de ses condensateurs dans les électrodes. Dans cet exemple, l'obtention de la commande CMD représente une confirmation par le stimulateur PM que l'arythmie détectée (F200) par le défibrillateur D est bien de type fibrillation ventriculaire. Le fait d'avoir déjà chargé (F350') les condensateurs permet au défibrillateur de gagner le temps nécessaire à leur recharge, et ainsi à mettre en œuvre l'étape de défibrillation F700' le plus rapidement possible.

Autrement, supposons que le compte à rebours TIMER a expiré au cours d'une étape F400' et que le défibrillateur D n'a pas obtenu de commande de défibrillation, au cours d'une étape F800, le défibrillateur D décharge ses condensateurs dans le corps de l'individu, lentement et progressivement afin de ne pas provoquer un choc électrique ou un danger pour la santé de l'individu.

Le défibrillateur D peut ne pas obtenir une commande car sa détection (F200) est erronée, le cœur n'ayant pas subit une fibrillation ventriculaire. Alternativement, l'arythmie détectée au cours de l'étape F200 peut bien correspondre à une fibrillation mais la stimulation E300 mise en œuvre par le stimulateur PM a suffi pour la traiter.

Dans le mode décrit en référence à la figure 13, lorsque le stimulateur PM envoie la commande CMD, il génère un signal clef de faible puissance et le propage, via l'une des sondes, par exemple la sonde S1, dans le corps. Ce signal clef étant interprétable par le défibrillateur D. Comme le défibrillateur D écoute (F100) en permanence l'activité électrique du cœur, il détecte le signal clef et en déduit la commande de défibrillation CMD.

A titre d'exemples, le signal clef peut avoir une forme sinusoïdale, carrée ou triangulaire.

Le stimulateur PM et le défibrillateur D peuvent être configurés pour se convenir de plusieurs types de signaux clefs, par exemple un signal clef pour une défibrillation après une durée déterminée, ou un signal clef pour un protocole de choc(s) adapté(s) à une famille donnée d'arythmies, etc.

Dans un autre mode, le stimulateur PM envoie la commande CMD directement au défibrillateur D, via un canal de communication radio ou autre moyen de communication.

Dans un mode de réalisation, le défibrillateur D n'écoute pas l'activité électrique du cœur. Il met en œuvre une défibrillation cardiaque sur obtention d'une commande générée par le stimulateur. La défibrillation ne dépend que de la commande obtenue.

Dans un mode de réalisation, lorsque le stimulateur PM détecte au cours de l'étape E200 une arythmie de type Tachycardie Supra-Ventriculaire (TSV), il n'effectue aucune stimulation cardiaque, mais reste en écoute (E100) de l'activité électrique du cœur.

Dans un mode de réalisation, en cas d'échec de la défibrillation cardiaque (F700') pour traiter l'arythmie détectée, plusieurs chocs de défibrillation peuvent être délivrés à la suite, par exemple avec une puissance croissante, tel qu'un premier choc de 5 J, suivi d'un deuxième choc de 20 J et si nécessaire d'un troisième choc de 40 J.

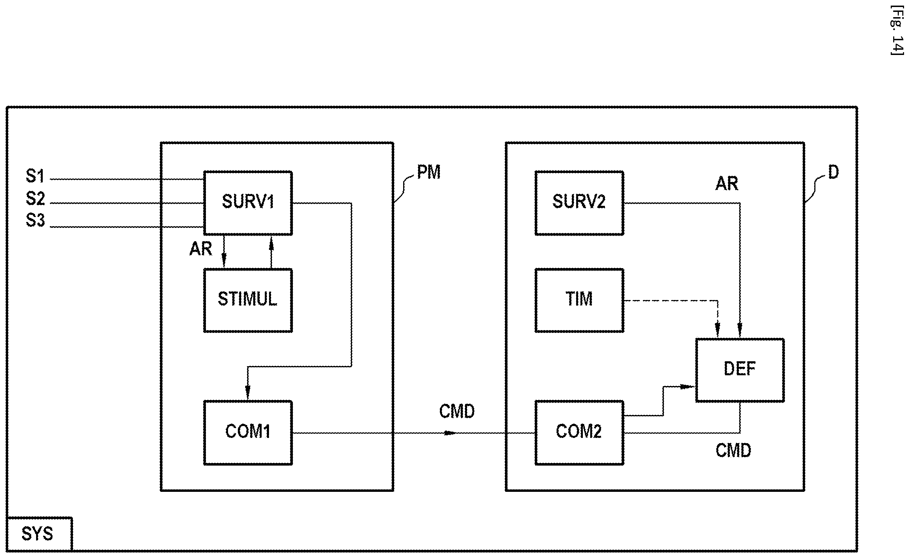

La **figure 14** représente des architectures fonctionnelles du système SYS de traitement d'au moins un problème cardiaque, du stimulateur PM et du défibrillateur D, selon le mode de réalisation décrit en référence aux figures 12 et 13.

Le système SYS comporte le stimulateur PM et le défibrillateur D, tous les deux pouvant être implantés dans un corps d'un individu, tels que présentés dans la figure 12.

Le stimulateur cardiaque PM comporte :
- au moins une sonde dédiée à la stimulation cardiaque, telles que les sondes S1, S2 et S3 ;
- un module d'écoute SURV1 configuré pour écouter (E100) l'activité électrique du cœur de l'individu via les sondes S1, S2 et S3, ce module SURV1 pouvant détecter (E200) une arythmie cardiaque ;
- un module de stimulation STIMUL configuré pour effectuer, sur détection (E200) d'une arythmie cardiaque, une stimulation cardiaque (E300) via les sondes en fonction de l'arythmie cardiaque détectée ; et
- un module de commande COM1 configuré pour envoyer (E500), sur détection (E400) de déficience de la stimulation, une commande de défibrillation cardiaque interprétable par le défibrillateur D.

Le défibrillateur cardiaque sous cutané D comporte une sonde comportant une électrode de défibrillation, et un boitier constituant une électrode de défibrillation et comportant:
- des condensateurs ;
- un module d'obtention d'une commande, COM2, configuré pour obtenir (F500, F500') une commande CMD de défibrillation générée par le stimulateur cardiaque PM, telle que la commande CMD ;
- un module de défibrillation DEF configuré pour effectuer (F700, F700'), sur obtention de ladite commande, une défibrillation cardiaque en fonction de la commande obtenue par une décharge des condensateurs dans les électrodes de défibrillation.

Ni le stimulateur PM ni le défibrillateur D ne comporte une sonde endocavitaire de défibrillation.

Dans le mode de réalisation décrit ici, le défibrillateur cardiaque D comporte en outre un module SURV2 d'écoute de l'activité électrique du cœur, ce module SURV2 étant configuré pour écouter (F100) l'activité électrique du cœur et détecter (F200) une arythmie cardiaque ; le module de défibrillation DEF étant configuré pour effectuer la défibrillation (F700, F700') en prenant en outre en considération l'arythmie cardiaque détectée.

Dans ce mode, le défibrillateur cardiaque D comporte en outre:
- un module TIM de déclenchement (F300') du compte à rebours temporel TIMER pour une durée d'attente déterminée, sur détection d'une arythmie cardiaque de type fibrillation ventriculaire; lorsque l'arythmie cardiaque détectée (F200) est une fibrillation ventriculaire, le module de défibrillation DEF est configuré pour :

- charger (F350') les condensateurs ;
- décharger (F800) les condensateurs dans le corps en absence d'obtention d'une commande de défibrillation à l'expiration (F400') de la durée d'attente; et
- n'effectuer la défibrillation cardiaque (F700') que sur obtention (F500') de la commande CMD avant l'expiration de la durée d'attente.

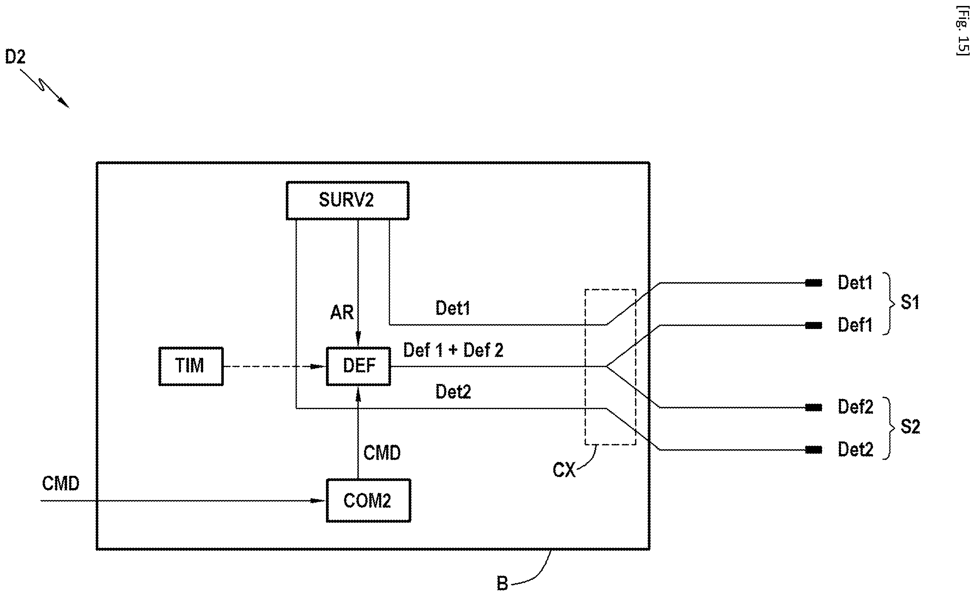

La **figure 15** illustre un défibrillateur D2, conforme au présent enseignement, selon un mode de réalisation. Le défibrillateur D2 diffère du défibrillateur D décrit précédemment en ce qu'il comporte deux sondes, S1 et S2, dont chacune comporte une électrode de défibrillation.

Le défibrillateur D2 comporte un boitier métallique B, par exemple en titane, contenant :
- des condensateurs ;
- un module d'obtention d'une commande, tel que le module COM2 du défibrillateur D décrit en référence à la figure 14 ;
- un module de défibrillation, tel que le module DEF du défibrillateur D décrit en référence à la figure 14 ;
- un module d'écoute de l'activité électrique du cœur, tel que le module SURV2 du défibrillateur D décrit en référence à la figure 14 ; et
- un bloc connecteur CX permettant de recevoir deux sondes sous-cutanées S1 et S2 avec une connectique bipolaire par exemple de type IS1.

Chacune des sondes S1 et S2 est connectée au boitier B et comprend :
- une électrode d'écoute ou de détection Det1, Det2 à son extrémité distale ;
- une électrode de défibrillation Def1, Def2 à son extrémité proximale ; et
- un connecteur bipolaire, par exemple de type IS1.

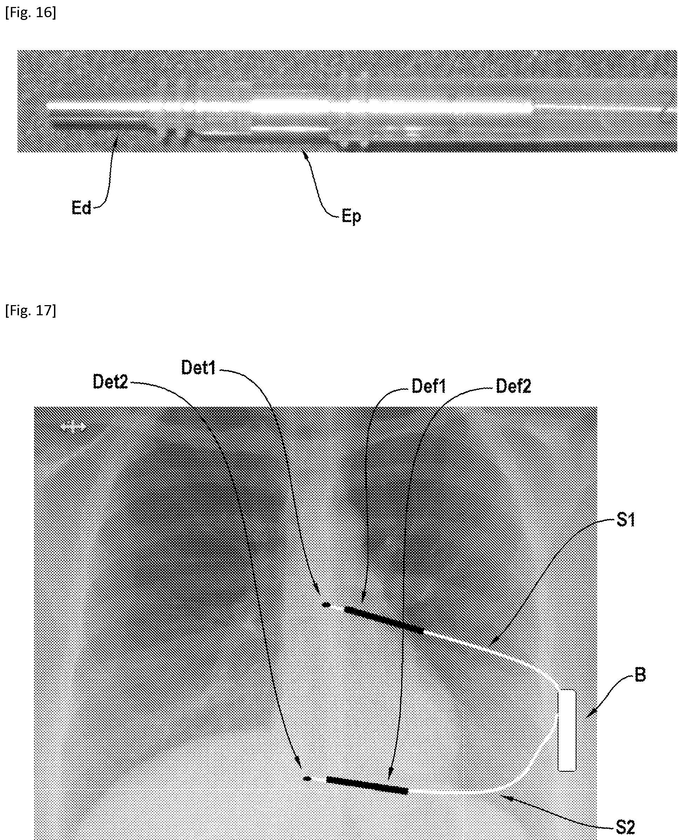

La **figure 16** illustre un connecteur bipolaire de type IS1 pour chacune des sondes S1 et S2. Ce connecteur du pôle proximal de la sonde S1 (ou S2) comporte une électrode distale longue Ed reliée à l'électrode de défibrillation Def1 (respectivement Def2), et une électrode annulaire proximale Ep reliée à l'électrode de détection Det1 (Det2).

La **figure 17** illustre un exemple d'implantation du défibrillateur D2. Le boitier B du défibrillateur D2 peut être implanté, comme le défibrillateur D, dans une loge latéro-postérieure thoracique gauche dans une loge pratiquée en position intermusculaire entre le muscle Grand Dorsal et le muscle Grand Dentelé, en regard de la pointe du ventricule gauche en radioscopie de face. La sonde S1 est canalisée entre la loge du boitier et l'appendice xyphoide en suivant un trajet longeant l'ombre de la base du cœur en radioscopie de face. L'extrémité distale de l'électrode Def1 se projette dans le prolongement du bord gauche du sternum. La sonde S2 est canalisée entre la loge du boitier et la face antérieure du sternum en suivant un trajet longeant l'arc moyen du cœur en radioscopie de face. L'extrémité distale de l'électrode Def2 se projette sur le bord gauche du sternum. La canalisation, appelée également la tunnelisation, est réalisée en utilisant un tunnelisateur (« tunneliser » en anglais) courbe portant un introducteur pelable.

Les deux sondes S1 et S2 sont fixées au plan musculaire profond de la loge du boitier à l'aide d'une olive de protection en silicone pour éviter leur rétraction dans la loge.

Les deux sondes S1 et S2 sont connectées au boitier B via le bloc connecteur CX et maintenus par une vis de serrage retenant l'électrode longue distale.

Les électrodes de détection Det1 et Det2 sont indépendantes l'une de l'autre. La détection du rythme cardiaque se fait en analysant un électrocardiogramme enregistré sur trois vecteurs : un vecteur reconstruit entre les électrodes de détection Det1 et Det2 ; un vecteur reconstruit entre l'électrode Det1 et le boitier ; et un vecteur reconstruit entre l'électrode Det2 et le boitier.

Les électrodes de défibrillation Def1 et Def2 sont branchées en parallèle et connectées à un pôle unique de défibrillation à l'intérieur du bloc connecteur CX. Le choc de défibrillation est délivré de façon simultanée :
- en sens direct : à partir des deux électrodes Def1 et Def2 (constituant une anode) en direction du boitier B (constituant une cathode) ; ou
- en sens inversé : à partir du boitier B (anode) en direction des deux électrodes Def1 et Def2 (cathode).

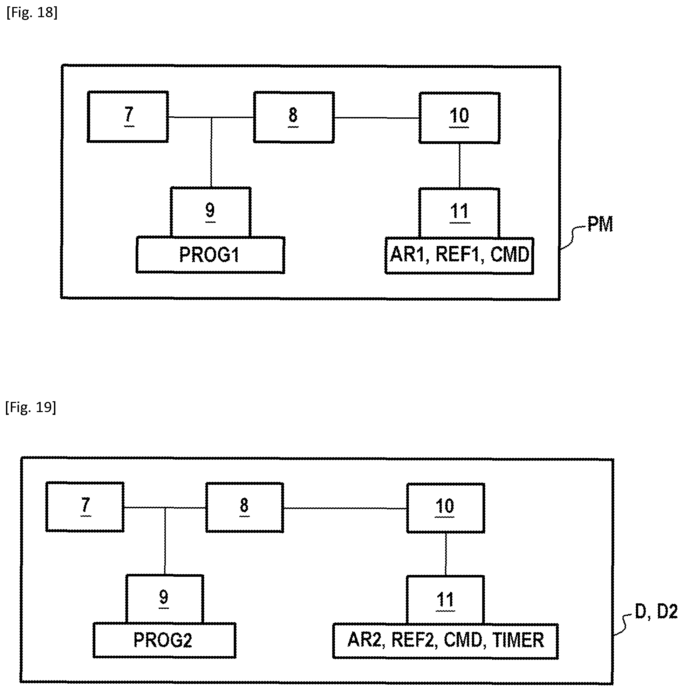

Dans le mode de réalisation décrit ici, le stimulateur cardiaque PM a l'architecture matérielle d'un ordinateur, telle qu'illustrée à la **figure 18****.**

L'architecture du stimulateur cardiaque PM comprend notamment un processeur 7, une mémoire vive 8, une mémoire morte 9, une mémoire flash non volatile 10 dans un mode particulier de réalisation, ainsi que des moyens de communication 11. De tels moyens sont connus en soi et ne sont pas décrits plus en détail ici.

La mémoire morte 9 du stimulateur cardiaque PM selon l'invention constitue un support d'enregistrement conforme à l'invention, lisible par le processeur 7 et sur lequel est enregistré ici un programme d'ordinateur PROG1 conforme au présent enseignement.

La mémoire 10 du stimulateur cardiaque PM permet d'enregistrer des variables utilisées pour l'exécution des étapes du procédé de stimulation cardiaque selon le ,présent enseignement telles qu'une information AR1 sur l'arythmie cardiaque détectée au cours de l'étape E200, un résultat REF1 de l'exécution des algorithmes diagnostiques pour identifier la pathologie correspondante à l'arythmie AR1, et la commande CMD.

Le programme d'ordinateur PROG1 définit des modules fonctionnels et logiciels ici, configurés pour effectuer une stimulation cardiaque, conformément au procédé de stimulation selon le présent enseignement. Ces modules fonctionnels s'appuient sur et/ou commandent les éléments matériels 7-11 du stimulateur cardiaque PM cités précédemment.

Dans le mode de réalisation décrit ici, le défibrillateur cardiaque D, D2, a l'architecture matérielle d'un ordinateur, telle qu'illustrée à la **figure 19****.**

L'architecture du défibrillateur cardiaque D, D2 comprend notamment un processeur 7, une mémoire vive 8, une mémoire morte 9, une mémoire flash non volatile 10 dans un mode particulier de réalisation, ainsi que des moyens de communication 11. De tels moyens sont connus en soi et ne sont pas décrits plus en détail ici.

La mémoire morte 9 du défibrillateur cardiaque D, D2 constitue un support d'enregistrement conforme au présent enseignement, lisible par le processeur 7 et sur lequel est enregistré ici une partie d'un programme d'ordinateur PROG2 conforme au présent enseignement.

La mémoire 10 du défibrillateur cardiaque D, D2 permet d'enregistrer des variables utilisées pour l'exécution des étapes du procédé de défibrillation cardiaque selon le présent enseignement, telles qu'une information AR2 sur l'arythmie cardiaque détectée au cours de l'étape F200, un résultat REF2 de l'exécution des algorithmes diagnostiques pour identifier la pathologie correspondante à l'arythmie AR2, la commande CMD, et la valeur du compte à rebours TIMER.

Le programme d'ordinateur PROG2 définit des modules fonctionnels et logiciels ici, configurés pour effectuer une défibrillation cardiaque, conformément au procédé de défibrillation selon le présent enseignement. Ces modules fonctionnels s'appuient sur et/ou commandent les éléments matériels 7-11 du défibrillateur cardiaque D, D2 cités précédemment. La présente invention est telle que définie dans les revendications suivantes.

## Revendications

1. Stimulateur cardiaque (PM) configuré pour être implanté dans le corps d'un individu et configuré pour communiquer avec un défibrillateur sous-cutané (D, D2), dont toutes les sondes sont sous-cutanées et implanté dans ledit corps dudit individu; ledit stimulateur cardiaque (PM) comportant:
- au moins une sonde dédiée à la stimulation cardiaque ;
- un module d'écoute (SURV1) configuré pour écouter l'activité électrique du cœur dudit individu via ladite au moins une sonde, ce module pouvant détecter une arythmie cardiaque ;
- un module de stimulation (STIMUL) configuré pour sur effectuer, sur détection d'une arythmie cardiaque, une stimulation cardiaque via ladite au moins une sonde en fonction de ladite arythmie cardiaque ; et
- un module de commande (COM1) configuré pour envoyer, sur détection de déficience de ladite stimulation, une commande de défibrillation cardiaque interprétable par le défibrillateur sous cutané (D, D2), dont toutes les sondes sont sous-cutanées, implanté dans ledit corps dudit individu.

2. Défibrillateur cardiaque sous cutané (D, D2) configuré pour être implanté dans le corps d'un individu et configuré pour opérer avec le stimulateur cardiaque (PM) selon la revendication précédente, ledit défibrillateur cardiaque (D, D2) comportant :
- au moins une sonde comportant une électrode de défibrillation, toutes les sondes dudit défibrillateur sous-cutané (D, D2) étant sous-cutanées ; et
- un boitier constituant une électrode de défibrillation et comportant :
- * des condensateurs ;
- * un module d'obtention d'une commande (COM2) configuré pour obtenir une commande de défibrillation générée par le stimulateur cardiaque (PM) selon la revendication précédente et implanté dans ledit corps ; et
- * un module de défibrillation (DEF) configuré pour effectuer, sur obtention de ladite commande, une défibrillation cardiaque en fonction de la commande obtenue par une décharge desdits condensateurs dans lesdites électrodes de défibrillation.

3. Défibrillateur cardiaque sous cutané (D, D2) selon la revendication 2 dans lequel ledit module de défibrillation (DEF) est configuré pour charger lesdits condensateurs sur obtention de ladite commande.

4. Défibrillateur cardiaque sous cutané (D, D2) selon l'une quelconque des revendications 2 ou 3 comportant en outre un module (SURV2) d'écoute de l'activité électrique du cœur dudit individu, comportant des électrodes d'écoute et étant configuré pour :
- écouter l'activité électrique du cœur ; et
- détecter une arythmie cardiaque,
ledit module de défibrillation (DEF) étant configuré pour effectuer ladite défibrillation en prenant en outre en compte ladite arythmie cardiaque détectée, ledit boitier constituant une dite électrode d'écoute, ladite au moins une sonde comportant au moins une dite électrode d'écoute.

5. Défibrillateur cardiaque sous cutané (D, D2) selon la revendication 4 comportant en outre:
- un module (TIM) de déclenchement d'un compte à rebours temporel pour une durée d'attente déterminée, sur détection d'une arythmie cardiaque de type fibrillation ventriculaire;
lorsque ladite arythmie cardiaque détectée est une fibrillation ventriculaire, le module de défibrillation est configuré pour :
- charger lesdits condensateurs ;
- décharger les condensateurs dans ledit corps en absence d'obtention d'une dite commande à l'expiration de ladite durée d'attente; et
- n'effectuer ladite défibrillation cardiaque que sur obtention de ladite commande avant l'expiration de ladite durée d'attente.

6. Défibrillateur cardiaque sous cutané (D2) selon l'une quelconque des revendications 2 à 5 comportant deux dites sondes sous-cutanées (S1, S2) dont chacune comporte une électrode de défibrillation (Def1, Def2), ledit module de défibrillation (DEF) étant configuré pour effectuer la défibrillation cardiaque en délivrant un choc de défibrillation de façon simultanée à partir desdites électrodes (Def1, Def2) des sondes (S1, S2) en direction du boitier (B), ou à partir du boitier (B) en direction desdites électrodes (Def1, Def2) des sondes (S1, S2).

7. Défibrillateur cardiaque sous cutané (D2) selon la revendication 6 dans lequel chacune desdites sondes sous-cutanées (S1, S2) comporte une dite électrode d'écoute (Det1, Det2), la détection du rythme cardiaque se fait en analysant un électrocardiogramme enregistré sur un vecteur reconstruit entre lesdites électrodes d'écoute (Det1, Det2), un vecteur reconstruit entre l'une desdites électrodes d'écoute (Det1) et le boitier (B), et un vecteur reconstruit entre l'autre électrode d'écoute (Det2) et le boitier (B).

8. Système de traitement d'au moins un problème cardiaque comportant :
- un stimulateur cardiaque selon la revendication 1 ; et
- un défibrillateur sous cutané (D, D2) selon l'une des revendications 2 à 7.

9. Système de traitement d'au moins un problème cardiaque selon la revendication 8 dans lequel :
- l'envoi d'une commande de défibrillation cardiaque par ledit module de commande (COM1) dudit stimulateur (PM) comporte une propagation d'un signal clef dans le corps dudit individu ; et
- l'obtention d'une commande de défibrillation par ledit module d'obtention d'une commande (COM2) dudit défibrillateur (D, D2) comporte une détection dudit signal clef, le défibrillateur comportant en le module (SURV2) d'écoute de l'activité électrique du cœur dudit individu selon l'une des revendications 4, 5 ou 7.

## Patentansprüche

1. Herzschrittmacher (PM), der so konfiguriert ist, dass er in den Körper eines Individuums implantiert wird, und so konfiguriert ist, dass er mit einem subkutanen Defibrillator (D, D2) kommuniziert, dessen Sonden alle subkutan und alle in den Körper des Individuums implantiert sind; wobei der Herzschrittmacher (PM) Folgendes umfasst: :
- mindestens eine Sonde, die der Herzstimulation gewidmet ist ;
- ein Abhörmodul (SURV1), das so konfiguriert ist, dass es die elektrische Aktivität des Herzens des Individuums über die mindestens eine Sonde abhört, wobei dieses Modul eine Herzarrhythmie erkennen kann ;
- ein Stimulationsmodul (STIMUL), das so konfiguriert ist, dass es in Reaktion auf die Erkennung einer Herzrhythmusstörung in Abhängigkeit von der Herzrhythmusstörung eine Herzstimulation über die mindestens eine Sonde durchführt; und
- ein Steuermodul (COM1), das so konfiguriert ist, dass es bei Erkennung eines Mangels der Stimulation einen Defibrillationsbefehl sendet, der von dem subkutanen Defibrillator (D, D2) interpretiert werden kann, dessen Sonden alle subkutan und alle in den Körper des Individuums implantiert sind.

2. Subkutaner Defibrillator (D, D2), der so konfiguriert ist, dass er in den Körper eines Individuums implantiert werden kann und so konfiguriert ist, dass er mit dem Herzschrittmacher (PM) nach dem vorhergehenden Anspruch arbeitet, wobei der Defibrillator (D, D2) Folgendes umfasst:
- mindestens eine Sonde, die eine Defibrillationselektrode umfasst, wobei alle Sonden des subkutanen Defibrillators (D, D2) subkutan sind; und
- ein Gehäuse, das eine Defibrillationselektrode bildet und Folgendes umfasst: :
- * Kondensatoren ;
- * ein Modul zum Erhalten eines Befehls (COM2), das so konfiguriert ist, dass es einen Defibrillationsbefehl erhält, der von dem Herzschrittmacher (PM) nach dem vorhergehenden Anspruch erzeugt wird und in den Körper implantiert ist; und
- * ein Defibrillationsmodul (DEF), das so konfiguriert ist, dass es bei Erhalt des Befehls eine Herzdefibrillation in Abhängigkeit von dem durch eine Entladung der Kondensatoren in den Defibrillationselektroden erhaltenen Befehl durchführt.

3. Subkutaner Herzdefibrillator (D, D2) nach Anspruch 2, bei dem das Defibrillationsmodul (DEF) so konfiguriert ist, dass es die Kondensatoren bei Erhalt des Befehls lädt.

4. Subkutaner Herzdefibrillator (D, D2) nach einem der Ansprüche 2 oder 3, der außerdem ein Modul (SURV2) zum Abhören der elektrischen Aktivität des Herzens des Individuums umfasst, das Abhörelektroden umfasst und so konfiguriert ist, dass es :
- auf die elektrische Aktivität des Herzens zu hören; und
- eine Herzrhythmusstörung zu erkennen,
wobei das Defibrillationsmodul (DEF) so konfiguriert ist, dass es die Defibrillation durchführt, indem es zusätzlich die erfasste Herzarrhythmie berücksichtigt, wobei das Gehäuse eine sogenannte Hörelektrode bildet, wobei die mindestens eine Sonde mindestens eine sogenannte Hörelektrode umfasst.

5. Subkutaner Kardioverter-Defibrillator (D, D2) nach Anspruch 4, der außerdem Folgendes umfasst:
- ein Modul (TIM) zum Auslösen eines zeitlichen Countdowns für eine bestimmte Wartezeit bei Erkennung einer Herzarrhythmie vom Typ Kammerflimmern;
wenn die erfasste Herzarrhythmie ein Kammerflimmern ist, das Defibrillationsmodul konfiguriert ist, um :
- die Kondensatoren zu laden ;
- die Kondensatoren in den Körper zu entladen, wenn nach Ablauf der Wartezeit kein Befehl erhalten wird; und
- die Defibrillation des Herzens nur dann durchzuführen, wenn der Befehl vor Ablauf der Wartezeit empfangen wird.

6. Subkutaner Herzdefibrillator (D2) nach einem der Ansprüche 2 bis 5, der zwei sogenannte subkutane Sonden (S1, S2) umfasst, von denen jede eine Defibrillationselektrode (Def1, Def2) umfasst, wobei das Defibrillationsmodul (DEF) so konfiguriert ist, dass es die Herzdefibrillation durchführt, indem es einen Defibrillationsschock gleichzeitig ausgehend von den Elektroden (Def1, Def2) der Sonden (S1, S2) in Richtung des Gehäuses (B) oder ausgehend von dem Gehäuse (B) in Richtung Elektroden (Def1, Def2) der Sonden (S1, S2) abgibt.

7. Subkutaner Defibrillator (D2) nach Anspruch 6, bei dem jede der subkutanen Sonden (S1, S2) eine sogenannte (Det1, Det2) umfasst, die Erfassung des Herzrhythmus durch Analyse eines Elektrokardiogramms erfolgt, das auf einem rekonstruierten Vektor zwischen den Hörelektroden (Det1, Det2) aufgezeichnet ist, Det2), einem rekonstruierten Vektor zwischen einer der Abhörelektroden (Det1) und dem Gehäuse (B) und einem rekonstruierten Vektor zwischen der anderen Abhörelektrode (Det2) und dem Gehäuse (B).

8. System zur Behandlung von mindestens einem Herzproblem, umfassend :
- einem Herzschrittmacher nach Anspruch 1; und
- einen subkutanen Defibrillator (D, D2) nach einem der Ansprüche 2 bis 7.

9. System zur Behandlung von mindestens einem Herzproblem nach Anspruch 8, wobei:
- das Senden eines Herzdefibrillationsbefehls durch das Steuermodul (COM1) des Herzschrittmachers (PM) eine Ausbreitung eines Schlüsselsignals im Körper des Individuums beinhaltet; und
- das Erhalten eines Defibrillationsbefehls durch das Modul zum Erhalten eines Befehls (COM2) des Defibrillators (D, D2) eine Erfassung des Schlüsselsignals umfasst, wobei der Defibrillator in dem Modul (SURV2) zum Abhören der elektrischen Aktivität des Herzens der Person nach einem der Ansprüche 4, 5 oder 7 umfasst.

## Claims

1. A pacemaker (PM) configured to be implanted in the body of an individual and configured to communicate with a subcutaneous defibrillator (D, D2), all of whose leads are subcutaneous and implanted in said body of said individual; said pacemaker (PM) comprising :
- at least one lead dedicated to cardiac stimulation ;
- a listening module (SURV1) configured to listen to the electrical activity of said individual's heart via said at least one lead, said module being able to detect cardiac arrhythmia;
- a stimulation module (STIMUL) configured to perform, upon detection of a cardiac arrhythmia, cardiac stimulation via said at least one probe as a function of said cardiac arrhythmia; and
- a control module (COM1) configured to send, upon detection of deficiency of said stimulation, a cardiac defibrillation command interpretable by the subcutaneous defibrillator (D, D2), all the leads of which are subcutaneous, implanted in said body of said individual.

2. A subcutaneous cardiac defibrillator (D, D2) configured to be implanted in the body of an individual and configured to operate with the pacemaker (PM) according to the preceding claim, said cardiac defibrillator (D, D2) comprising :
- at least one lead comprising a defibrillation electrode, all the leads of said subcutaneous defibrillator (D, D2) being subcutaneous; and
- a housing forming a defibrillation electrode and comprising :
- * capacitors ;
- * a command obtaining module (COM2) configured to obtain a defibrillation command generated by the pacemaker (PM) according to the preceding claim and implanted in said body; and
- * a defibrillation module (DEF) configured to perform, upon obtaining said command, cardiac defibrillation as a function of the command obtained by discharging said capacitors in said defibrillation electrodes.

3. A subcutaneous cardiac defibrillator (D, D2) according to claim 2 wherein said defibrillation module (DEF) is configured to charge said capacitors on receipt of said command.

4. A subcutaneous cardiac defibrillator (D, D2) according to any one of claims 2 or 3 further comprising a module (SURV2) for listening to the electrical activity of the heart of said individual, comprising listening electrodes and being configured to:
- listen to the electrical activity of the heart; and
- detect cardiac arrhythmia,
said defibrillation module (DEF) being configured to perform said defibrillation by further taking into account said detected cardiac arrhythmia, said housing forming a said listening electrode, said at least one probe comprising at least one said listening electrode.

5. A subcutaneous cardiac defibrillator (D, D2) according to claim 4 further comprising:
- a module (TIM) for triggering a time countdown for a predetermined waiting time, upon detection of a cardiac arrhythmia of the ventricular fibrillation type;
when said detected cardiac arrhythmia is ventricular fibrillation, the defibrillation module is configured to :
- charge said capacitors ;
- discharge the capacitors into said body in the absence of obtaining a said command on expiry of said waiting time; and
- perform said cardiac defibrillation only on receipt of said command prior to expiry of said waiting time.

6. Subcutaneous cardiac defibrillator (D2) according to any one of claims 2 to 5 comprising two said subcutaneous probes (S1, S2) each of which comprises a defibrillation electrode (Def1, Def2), said defibrillation module (DEF) being configured to perform cardiac defibrillation by delivering a defibrillation shock simultaneously from said electrodes (Def1, Def2) of the probes (S1, S2) towards the housing (B), or from the housing (B) towards said electrodes (Def1, Def2) of the probes (S1, S2).

7. Subcutaneous cardiac defibrillator (D2) according to claim 6 in which each of said subcutaneous leads (S1, S2) comprises a said listening electrode (Det1, Det2), the detection of the cardiac rhythm is done by analyzing an electrocardiogram recorded on a vector reconstructed between said listening electrodes (Det1, Det2), a vector reconstructed between one of said listening electrodes (Det1) and the housing (B), and a vector reconstructed between the other listening electrode (Det2) and the housing (B).

8. A system for treating at least one cardiac problem comprising :
- a pacemaker as claimed in claim 1; and
- a subcutaneous defibrillator (D, D2) according to one of claims 2 to 7.

9. A system for treating at least one cardiac problem according to claim 8 wherein :
- sending a cardiac defibrillation command by said command obtaining module (COM1) of said pacemaker (PM) comprises propagation of a key signal in the body of said individual; and
- obtaining a defibrillation command by said command obtaining module (COM2) of said defibrillator (D, D2) comprises a detection of said key signal, the defibrillator comprising in the module (SURV2) for listening to the electrical activity of the heart of said individual according to one of claims 4, 5 or 7.
